# EUROPEAN PATENT APPLICATION

(11) **EP 2 327 788 A1**
(43) Date of publication of application: **01.06.2011**
(21) Application number: 09014709.1
(22) Date of filing: 25.11.2009
(51) Int. Cl.: C12N 15/90, C12N 15/85, A01K 67/027

(54) **Conditional expression of transgenes in vivo**

(71) Applicant: Helmholtz Zentrum München Deutsches Forschungszentrum für Gesundheit und Umwelt (GmbH), 85764 Neuherberg (DE); University of Frankfurt Medical School, 60590 Frankfurt am Main (DE)
(72) Inventor: Schnütgen, Frank, 63755 Alzenau (DE); Schebelle, Laura, 85435 Erding (DE); Floss, Thomas, 81739 München (DE)
(74) Representative: Vossius & Partner

(57) **Abstract**

The present invention relates to a method of producing a cell comprising a conditionally active transgene in its genome, the method comprising (a) introducing into the cell a targeting vector, wherein the targeting vector comprises (i) a 5' recombinase recognition site specifically recognised by a first recombinase, wherein the first recombinase is endogenously present in the cell or wherein the first recombinase or a nucleic acid molecule encoding said first recombinase in expressible form is introduced into the cell; followed by (ii) a 5' recombinase recognition site specifically recognised by a second recombinase, wherein the second recombinase is not endogenously present or is not active in the cell; followed by (iii) a selection cassette comprising a positively selectable marker gene; followed by (iv) a 3' recombinase recognition site specifically recognised by a third recombinase, wherein the third recombinase is not endogenously present or is not active in the cell; followed by (v) the transgene; followed by (vi) a 3' recombinase recognition site specifically recognised by a fourth recombinase, wherein the fourth recombinase is endogenously present in the cell or wherein the fourth recombinase or a nucleic acid molecule encoding said fourth recombinase in expressible form is introduced into the cell; wherein the genome of the cell comprises a 5' recombinase recognition site and a 3' recombinase recognition site that are identical to the recombinase recognition sites of (i) and (vi), and wherein said recombinase recognition sites comprised in the genome of the cell are located 3' of an endogenous cellular promoter such that introduction of the targeting vector into the genome by site specific recombination results in the promoter being operatively linked to the selectable marker gene; and (b) culturing the cell in the presence of a selection medium specific for the selectable marker encoded by the selectable marker gene of (iii). The present invention further relates to a method of producing a conditional transgenic non-human mammalian animal as well as to a conditional transgenic non-human mammalian animal obtainable by said method. The present invention also relates to a transgenic TDP-43 mouse, comprising a transgenic cassette in intron 1 of the mouse Tardbp gene.

SEQUENCE LISTING

<110> Helmholtz Zentrum München

<120> Conditional expression of transgenes in vivo

<130> R2951 EP

<160> 41

<170> PatentIn version 3.3

<210> 1
<211> 30
<212> DNA
<213> artificial sequence

<220>
<223> /note="Description of artificial sequence: primer"

<400> 1
atgcggccgc gccaccatga aaaagcctga 30

<210> 2
<211> 30
<212> DNA
<213> artificial sequence

<220>
<223> /note="Description of artificial sequence: primer"

<400> 2
atgcggccgc aagcttctga tggaattaga 30

<210> 3
<211> 29
<212> DNA
<213> artificial sequence

<220>
<223> /note="Description of artificial sequence: forward primer"

<400> 3
ataagcttac catggcctcc tccgaggac 29

<210> 4
<211> 29
<212> DNA
<213> artificial sequence

<220>
<223> /note="Description of artificial sequence: reverse primer"

<400> 4
atgagctcct acaggaacag gtggtggcg 29

<210> 5
<211> 20
<212> DNA
<213> artificial sequence

<220>
<223> /note="Description of artificial sequence: primer"

<400>  5
gccggaagag atgctgagtc                                                   20


<210>  6
<211>  22
<212>  DNA
<213>  artificial sequence

<220>
<223>  /note="Description of artificial sequence: primer"

<400>  6
tacccgtgta tccaataaac cc                                                22


<210>  7
<211>  23
<212>  DNA
<213>  artificial sequence

<220>
<223>  /note="Description of artificial sequence: primer"

<400>  7
aaactggttt tcattgggga tca                                               23


<210>  8
<211>  22
<212>  DNA
<213>  artificial sequence

<220>
<223>  /note="Description of artificial sequence: primer"

<400>  8
tacccgtgta tccaataaac cc                                                22


<210>  9
<211>  22
<212>  DNA
<213>  artificial sequence

<220>
<223>  /note="Description of artificial sequence: primer"

<400>  9
tcccccatgt ttctgtaatt gg                                                22


<210>  10
<211>  23
<212>  DNA
<213>  artificial sequence

<220>
<223>  /note="Description of artificial sequence: primer"

<400>  10
gccaaaccta caggtgggtc ttt                                               23


<210>  11
<211>  20
<212>  DNA
<213>  artificial sequence

<220>
<223>  /note="Description of artificial sequence: primer"

<400>  11
atcgaatgta gcccaggatg                                                    20


<210>  12
<211>  23
<212>  DNA
<213>  artificial sequence

<220>
<223>  /note="Description of artificial sequence: primer"

<400>  12
gccaaaccta caggtgggtc ttt                                                23


<210>  13
<211>  25
<212>  DNA
<213>  artificial sequence

<220>
<223>  /note="Description of artificial sequence: primer"

<400>  13
catcttgaac ctcaagtttg ccttt                                              25


<210>  14
<211>  22
<212>  DNA
<213>  artificial sequence

<220>
<223>  /note="Description of artificial sequence: primer"

<400>  14
tacccgtgta tccaataaac cc                                                 22


<210>  15
<211>  22
<212>  DNA
<213>  artificial sequence

<220>
<223>  /note="Description of artificial sequence: primer"

<400>  15
ctggcttcct cccacttgtg tt                                                 22


<210>  16
<211>  22
<212>  DNA
<213>  artificial sequence

<220>
<223>  /note="Description of artificial sequence: primer"

<400>  16
tacccgtgta tccaataaac cc                                                 22

<210> 17
<211> 22
<212> DNA
<213> artificial sequence

<220>
<223> /note="Description of artificial sequence: primer"

<400> 17
agaggtgacc ctgctggaaa tg 22


<210> 18
<211> 22
<212> DNA
<213> artificial sequence

<220>
<223> /note="Description of artificial sequence: primer"

<400> 18
tacccgtgta tccaataaac cc 22


<210> 19
<211> 22
<212> DNA
<213> artificial sequence

<220>
<223> /note="Description of artificial sequence: primer"

<400> 19
ccaaggtggg aaagatgagg tg 22


<210> 20
<211> 23
<212> DNA
<213> artificial sequence

<220>
<223> /note="Description of artificial sequence: primer"

<400> 20
gccaaaccta caggtgggtc ttt 23


<210> 21
<211> 22
<212> DNA
<213> artificial sequence

<220>
<223> /note="Description of artificial sequence: primer"

<400> 21
cacatggtga ccttcagagc ag 22


<210> 22
<211> 23
<212> DNA
<213> artificial sequence

<220>
<223> /note="Description of artificial sequence: primer"

<400>  22
gccaaaccta caggtgggtc ttt                                              23


<210>  23
<211>  25
<212>  DNA
<213>  artificial sequence

<220>
<223>  /note="Description of artificial sequence: primer"

<400>  23
acaggctacc gtatttcgta accaa                                            25


<210>  24
<211>  23
<212>  DNA
<213>  artificial sequence

<220>
<223>  /note="Description of artificial sequence: primer"

<400>  24
gctagcttgc caaatacagg tgg                                              23


<210>  25
<211>  24
<212>  DNA
<213>  artificial sequence

<220>
<223>  /note="Description of artificial sequence: primer"

<400>  25
gccaaaccta caggtggggt cttt                                             24


<210>  26
<211>  21
<212>  DNA
<213>  artificial sequence

<220>
<223>  /note="Description of artificial sequence: primer"

<400>  26
tgtaaaacga cgggatccgc c                                                21


<210>  27
<211>  21
<212>  DNA
<213>  artificial sequence

<220>
<223>  /note="Description of artificial sequence: primer"

<400>  27
ctccgcctcc tcttcctcca t                                                21


<210>  28
<211>  22
<212>  DNA
<213>  artificial sequence

<220>
<223>  /note="Description of artificial sequence: primer"

<400>  28
cctcccccgt gccttccttg ac                                                22


<210>  29
<211>  22
<212>  DNA
<213>  artificial sequence

<220>
<223>  /note="Description of artificial sequence: primer"

<400>  29
tttgagggga cgacgacagt at                                                22


<210>  30
<211>  24
<212>  DNA
<213>  artificial sequence

<220>
<223>  /note="Description of artificial sequence: primer"

<400>  30
gccaaaccta caggtggggt cttt                                              24


<210>  31
<211>  23
<212>  DNA
<213>  artificial sequence

<220>
<223>  /note="Description of artificial sequence: primer"

<400>  31
atcaaggaaa ccctggacta ctg                                               23


<210>  32
<211>  23
<212>  DNA
<213>  artificial sequence

<220>
<223>  /note="Description of artificial sequence: primer"

<400>  32
atcaaggaaa ccctggacta ctg                                               23


<210>  33
<211>  24
<212>  DNA
<213>  artificial sequence

<220>
<223>  /note="Description of artificial sequence: primer"

<400>  33
gccaaaccta caggtggggt cttt                                              24

<210> 34
<211> 22
<212> DNA
<213> artificial sequence

<220>
<223> /note="Description of artificial sequence: primer"

<400> 34
aatgtttatc ggtggactga gc 22


<210> 35
<211> 20
<212> DNA
<213> artificial sequence

<220>
<223> /note="Description of artificial sequence: primer"

<400> 35
cgtttcgttg tgggatctct 20


<210> 36
<211> 22
<212> DNA
<213> artificial sequence

<220>
<223> /note="Description of artificial sequence: primer"

<400> 36
gtgcttcacg tacaccttgg ag 22


<210> 37
<211> 23
<212> DNA
<213> artificial sequence

<220>
<223> /note="Description of artificial sequence: primer"

<400> 37
atgcccaaga agaagaggaa ggt 23


<210> 38
<211> 25
<212> DNA
<213> artificial sequence

<220>
<223> /note="Description of artificial sequence: primer"

<400> 38
gaaatcagtg cgttcgaacg ctaga 25


<210> 39
<211> 24
<212> DNA
<213> artificial sequence

<220>
<223> /note="Description of artificial sequence: primer"

<400>  39
caagctctga tagagttggt caag                                              24


<210>  40
<211>  22
<212>  DNA
<213>  artificial sequence

<220>
<223>  /note="Description of artificial sequence: primer"

<400>  40
cctcccccgt gccttccttg ac                                                22


<210>  41
<211>  21
<212>  DNA
<213>  artificial sequence

<220>
<223>  /note="Description of artificial sequence: primer"

<400>  41
ctccgcctcc tcttcctcca t                                                 21

## Description

The present invention relates to a method of producing a cell comprising a conditionally active transgene in its genome, the method comprising (a) introducing into the cell a targeting vector, wherein the targeting vector comprises (i) a 5' recombinase recognition site specifically recognised by a first recombinase, wherein the first recombinase is endogenously present in the cell or wherein the first recombinase or a nucleic acid molecule encoding said first recombinase in expressible form is introduced into the cell; followed by (ii) a 5' recombinase recognition site specifically recognised by a second recombinase, wherein the second recombinase is not endogenously present or is not active in the cell; followed by (iii) a selection cassette comprising a positively selectable marker gene; followed by (iv) a 3' recombinase recognition site specifically recognised by a third recombinase, wherein the third recombinase is not endogenously present or is not active in the cell; followed by (v) the transgene; followed by (vi) a 3' recombinase recognition site specifically recognised by a fourth recombinase, wherein the fourth recombinase is endogenously present in the cell or wherein the fourth recombinase or a nucleic acid molecule encoding said fourth recombinase in expressible form is introduced into the cell; wherein the genome of the cell comprises a 5' recombinase recognition site and a 3' recombinase recognition site that are identical to the recombinase recognition sites of (i) and (vi), and wherein said recombinase recognition sites comprised in the genome of the cell are located 3' of an endogenous cellular promoter such that introduction of the targeting vector into the genome by site specific recombination results in the promoter being operatively linked to the selectable marker gene; and (b) culturing the cell in the presence of a selection medium specific for the selectable marker encoded by the selectable marker gene of (iii). The present invention further relates to a method of producing a conditional transgenic non-human mammalian animal as well as to a conditional transgenic non-human mammalian animal obtainable by said method. The present invention also relates to a transgenic TDP-43 mouse, comprising a transgenic cassette in intron 1 of the mouse Tardbp gene.

In this specification, a number of documents including patent applications and manufacturer's manuals is cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

A number of different strategies for the modification of the genome, and in particular the mouse genome, have been investigated so far. One exemplary aspect is the introduction of disease-mediating mutations or entire disease-mediating genes into the genome of an animal model. Most of the methods for achieving this involve the introduction of transgenes into the genome as well as the use of homologous recombination (HR) techniques for targeted gene modifications or the use of non-targeted gene trapping.

For the generation of traditional transgenic animals, genes responsible for particular traits or disease susceptibilities are chosen and extracted and are injected into fertilized mouse eggs. Embryos are implanted in the uterus of a surrogate mother and the selected genes will be expressed by some of the offspring. These conventional transgenic approaches offer the advantage that they are relatively straightforward and inexpensive. In addition, high levels of target gene expression can be achieved, and transgenic overexpressing animals, such as for example mice, often demonstrate obvious phenotypes. However, the site of integration as well as the copy number of the transgene in the genome can seriously affect tissue specificity and levels of transgene expression (Schonig et al., 2002). In particular, the site of integration is generally random, thus not allowing for a targeted modification of the genome. As a consequence, a number of founder lines may need to be screened, which is cost- and time-consuming. The use of insulator sequences to protect the transgene expression from effects of integration site have so far provided only variable success (Truffinet et al., 2005). However, even well-characterized promoters are often expressed at lower levels in non-target tissues, so rigorous analysis should include examination of transgene expression in a range of tissues. In addition, over-expression models generally produce non-physiological levels of the gene and corresponding protein expression, thus deviating from the naturally occurring expression patterns of the gene of interest and rendering the model potentially unsuitable.

A further disadvantage of using traditional transgenic approaches lies in the fact that the modification of the genome, once it has been effected, is generally irreversible and cannot be further controlled by the scientist.

In contrasted to the non-targeted transgenic approaches, gene modification via homologous recombination is based on the targeted insertion of a selectable marker (often the neomycin phosphotransferase gene, neo) into an exon of the target gene, the replacement of one or more exons or, alternatively, the insertion of additional nucleic acid sequences into a target locus. The mutant allele is initially assembled in a specifically designed gene targeting vector such that the sequence to be inserted is flanked at both sides with genomic segments of the target gene that serve as homology regions to initiate homologous recombination. Using such standard gene targeting vectors the efficiency at which homologous recombinant ES cell clones are obtained is the range of 0.1% to 10%. This rate depends on the length of the vector homology region, the degree of sequence identity of this region with the genomic DNA and likely on the differential accessibility of individual genomic loci to homologous recombination. Upon the isolation of recombinant ES cell clones, modified ES cells are injected into blastocysts to transmit the mutant allele through the germ line of chimeras and to establish a mutant strain. (Hasty P, Abuin A, Bradley A., 2000, In Gene Targeting: a practical approach, ed. AL Joyner, pp. 1-35. Oxford: Oxford University Press; Nagy A, Gertsenstein M, Vintersten K, Behringer R., 2003. Manipulating the Mouse Embryo. Cold Spring Harbour, New York: Cold Spring Harbour Laboratory Press).

To avoid embryonic lethality and to study gene function only in specific cell types, conditional gene targeting schemes allow gene inactivation in specific cell types or developmental stages. In conditional mutants, gene inactivation is achieved by the insertion of two recombinase recognition sites (RRS) for a site-specific DNA recombinase into introns of the target gene such that recombination results in the deletion of the RRS-flanked exons. Conditional mutants require the generation of two mouse strains: one strain harbouring a RRS flanked gene segment obtained by gene targeting in ES cells and a second, transgenic strain expressing the corresponding recombinase in one or several cell types. The conditional mutant is generated by crossing these two strains such that target gene inactivation occurs in a spatial and temporal restricted manner, according to the pattern of recombinase expression in the second transgenic strain (Nagy A, Gertsenstein M, Vintersten K, Behringer R. 2003. Manipulating the Mouse Embryo, third edition ed. Cold Spring Harbour, New York: Cold Spring Harbour Laboratory Press; Torres RM, Kühn R. 1997. Laboratory protocols for conditional gene targeting. Oxford: Oxford University Press). Conditional mutants have been used to address various biological questions which could not be resolved with germ line mutants, often because a null allele results in an embryonic or neonatal lethal phenotype.

Although the generation of mouse mutants via genome engineering in ES cells and the derivation of germ line transmitting chimeras is established as a routine procedure, this approach typically requires a large amount of work, in particular for vector construction. In addition, one problem that is encountered during these procedures is the low efficiency of homologous recombination in ES cells. Therefore, the successful generation of targeted knockout or transgenic mice is time- and cost intensive.

Gene trapping, on the other hand, is a high-throughput approach that is used to introduce insertional mutations across the genome in cells. This method traditionally relied on random integration into chromosomal loci, which selects actively expressed genes. Recently, a number of techniques have been developed to achieve insertion of nucleic acid sequences of interest into pre-characterised loci.

Baer and Bode, 2001 provide an overview over the principle underlying the Cre/loxP and Flp/FRT systems and discuss the recombinase mediated cassette exchange (RMCE) approach as a tool for site-specific integrations of nucleic acid sequences into a host genome. The authors highlight that it is most desirable to introduce cassettes that do not comprise any additional sequences other than the gene of interest and that no selection marker is present in the targeted locus. If positive selection is nonetheless required for achieving higher efficiency, then the authors conclude that it has to be ensured that the selection is of a kind and in a position where it will not interfere with the gene of interest.

Cesari *et al.* 2004 describe the use of a Flp RMCE technique. The technique was employed to replace a cassette that had previously been introduced into a gene locus in order to create a knock-out phenotype. The authors show that it is possible to successfully exchange one cassette with a different cassette and conclude that RMCE provides an elegant tool for studying the functions of previously tagged genes.

Cobellis *et al.* 2005 describe exchange vectors that allow for the insertion of nucleic acid sequences of interest into trapped loci using RMCE. The exchange cassettes described include a nucleic acid sequence of interest and a selection marker gene, in opposite directions. Expression of the nucleic acid sequence of interest and the selection marker gene are independent of each other. Upon integration into the genome, the nucleic acid sequence of interest is expressed as a result of activation of the endogenous promoter of the trapped gene. The selection marker gene, on the other hand, is expressed from its own promoter, which is part of the cassette. Thus, expression of both the nucleic acid sequence of interest and the selection marker gene cannot be modified further after successful integration into the genome. The use of a non-removable TK promoter-driven antibiotic resistance cassette selected a relatively high number of random insertions and had an exchange efficiencies of a relatively low frequency of 7%.

EP0939120 describes a method of marker-free DNA expression cassette exchange in the genome of cells or parts of cells. A first cassette comprising a positive-negative selection marker flanked by heterotypic FRT sites is introduced into the genome by homologous recombination or random integration. After positive selection, the first cassette is exchanged by Flp recombinase mediated cassette exchange against a second, marker-less cassette. Clones containing the desired exchange cassette are obtained by negative selection.

WO2006/056617 describes a conditional knock-out method based on a gene trap cassette that employs two directional site-specific recombination systems which serve to invert the gene trap cassette between a mutagenic orientation on the sense strand and a non-mutagenic orientation on the anti-sense strand. The method allows for inactivating genes in the same manner as traditional gene trap methods, by introducing a nucleic acid sequence comprising a poly-A sequence and, thus, terminating expression of an endogenous gene. In addition, the method allows for conditionally switching said inactivation on and off by inverting the sequence between the sense and anti-sense strand. At the same time, however, the method is restricted to the inactivation of endogenous genes.

While demonstrating the enormous developments in this field, the above described methods do not provide for conditional expression, i.e. activation, of transgenic nucleic acid sequence of interest after integration into the genome. The technical problem underlying the present invention is thus the provision of improved methods of producing cells and transgenic animals comprising a conditionally active transgene in its genome.

The solution to this technical problem is achieved by providing the embodiments characterised in the claims.

Accordingly, the present invention relates to a method of producing a cell comprising a conditionally active transgene in its genome, the method comprising (a) introducing into the cell a targeting vector, wherein the targeting vector comprises (i) a 5' recombinase recognition site specifically recognised by a first recombinase, wherein the first recombinase is endogenously present in the cell or wherein the first recombinase or a nucleic acid molecule encoding said first recombinase in expressible form is introduced into the cell; followed by (ii) a 5' recombinase recognition site specifically recognised by a second recombinase, wherein the second recombinase is not endogenously present or is not active in the cell; followed by (iii) a selection cassette comprising a positively selectable marker gene; followed by (iv) a 3' recombinase recognition site specifically recognised by a third recombinase, wherein the third recombinase is not endogenously present or is not active in the cell; followed by (v) the transgene; followed by (vi) a 3' recombinase recognition site specifically recognised by a fourth recombinase, wherein the fourth recombinase is endogenously present in the cell or wherein the fourth recombinase or a nucleic acid molecule encoding said fourth recombinase in expressible form is introduced into the cell; wherein the genome of the cell comprises a 5' recombinase recognition site and a 3' recombinase recognition site that are identical to the recombinase recognition sites of (i) and (vi), and wherein said recombinase recognition sites comprised in the genome of the cell are located 3' of an endogenous cellular promoter such that introduction of the targeting vector into the genome by site specific recombination results in the promoter being operatively linked to the selectable marker gene; and (b) culturing the cell in the presence of a selection medium specific for the selectable marker encoded by the selectable marker gene of (iii).

In accordance with the present invention, the cell is selected from the group consisting of an embryonic stem cell, an induced pluripotent stem cell (iPS), a primordial germ cell or a somatic cell.

The term "embryonic stem cells", as used throughout the present invention, refers to stem cells derived from the inner cell mass of an early stage embryo known as a blastocyst. Embryonic stem (ES) cells are pluripotent, i.e. they are able to differentiate into all derivatives of the three primary germ layers: ectoderm, endoderm, and mesoderm. Recent advances in embryonic stem cell research have led to the possibility of creating new embryonic stem cell lines without destroying embryos, for example by using a single-cell biopsy similar to that used in preimplantation genetic diagnosis (PGD), which does not interfere with the embryo's developmental potential (Klimanskaya *et al.* (2006)). Furthermore, a large number of established embryonic stem cell lines are available in the art (according to the U.S. National Institutes of Health, 21 lines are currently available for distribution to researchers), thus making it possible to work with embryonic stem cells without the necessity to destroy an embryo. In a preferred embodiment, the embryonic stem cells are non-human embryonic stem cells.

"Induced pluripotent stem (iPS) cells", in accordance with the present invention, are pluripotent stem cell derived from a non-pluripotent cell, typically an adult somatic cell, by inducing a "forced" expression of certain genes. Induced pluripotent stem cells are identical to natural pluripotent stem cells, such as embryonic stem cells in many respects, such as the expression of certain stem cell genes and proteins, chromatin methylation patterns, doubling time, embryoid body formation, teratoma formation, viable chimera formation, and potency and differentiability. Induced pluripotent stem cells are an important advancement in stem cell research, as they allow researchers to obtain pluripotent stem cells without the use of embryos (Nishikawa *et al.* (2008)). The induced pluripotent stem cells may be obtained from any adult somatic cells, preferably from fibroblasts, e.g. from skin tissue biopsies.

The term "primordial germ cells", as used herein, refers to precursor germ cells which have not yet reached the gonads where they mature into sperm or ova as well as to mature spermatozoa and ova. Methods for the culturing of primordial germ cells including suitable media are well established in the art. For example, primordial germ cells may be differentiated *in vitro* from ES cells, such as for example the above recited ES cells and established ES cell lines. Also iPS cells can be used as a starting cell for the differentiation of primordial germ cells (Park *et al.* 2009) In a preferred embodiment, the primordial germ cell are non-human primordial germ cell.

The term "somatic cells", as used herein, refers to any cell type in the mammalian body apart from germ cells and undifferentiated or partially differentiated stem cells. Preferably, the somatic cell is a cell from which induced pluripotent stem cells can be derived.

The term "conditionally active transgene", in accordance with the present invention, refers to a nucleic acid sequence that has been introduced into the genome in such a way that it is not expressed constitutively. Only upon recombinase-mediated removal of the selection marker cassette, which is located 5' of the transgene, does the transgene become expressed by means of the endogenous promoter. It is therefore possible to activate transgene expression in a time- and tissue-specific manner, for example by introducing the second and third recombinase into the cell or by crossing a transgenic animal obtained from a cell produced by the method of the invention with a second transgenic animal expressing the respective recombinase(s) in the tissue and/or at the time of interest.

The term "transgene", in accordance with the present invention, refers to a nucleic acid sequence that is introduced into the genome of the cell. In non-limiting examples, the transgene can consist of an exogenous gene not normally present in the target sequence, such as for example a gene from one species that is introduced into a cell derived from another species. In a further non-limiting example, the transgene can be essentially identical to the part of the genome but carrying a disease-causing mutation or, alternatively, the transgene can be a gene compensating for the lack of a gene. The terms "nucleic acid molecules" as well as "nucleic acid sequences", as used throughout the present description, are used according to the definitions provided in the art and include DNA, such as cDNA or genomic DNA, and RNA, such as mRNA.

The term "targeting vector" in accordance with the present invention refers to a vector that comprises the nucleic acid sequences that are to be integrated into the genome of the cell as well as the elements that are required to enable site-specific recombination. The targeting vector comprises at least the elements defined in (i) to (vi) in the order listed (also referred to as the transgenic cassette herein), either in the 5' to 3' direction or in the 3' to 5' direction. The term "followed by" as used in accordance with the present invention is meant to define the order of the respective elements of the targeting vector. This term encompasses that an element is immediately followed by the next element but, additionally, also encompasses the presence of spacer regions between elements, such that an element is not immediately followed by next element.

The targeting vector therefore comprises the transgene (v) that is to be introduced into the genome of the cell. Further comprised is a selection cassette (iii) flanked by two recombinase recognition sequences (ii) and (iv), for which no recombinase or nucleic acid molecule coding therefore is present or active in the cell.

Preferably, the selection cassette does not comprise a promoter. In this case, the selection cassette serves to express the selectable marker only upon insertion of the cassette behind an endogenous promoter present in the cellular genome, which enhances the selection of cells with successful integration of the targeting vector. The elements (ii) to (v) are further flanked by two recombinase recognition sequences (i) and (vi), for which the respective recombinase is endogenously present in the cell or is introduced into the cell. Alternatively, a nucleic acid molecule encoding the enzyme may be introduced into the cell. The term "a nucleic acid molecule encoding said first/fourth recombinase in expressible form" refers to a nucleic acid molecule which, upon expression in the cell, results in the functional recombinase protein.

The targeting vector can e.g. be synthesized by standard methods, however, parts of the vector, such as for example the transgene, can also be isolated from natural sources and ligated with the remaining parts of the targeting vector using techniques known in the art. The introduction of the targeting vector into the cell may be achieved using any of the methods known in the art for introducing nucleic acid molecules into cells. Such methods include for example calcium phosphate-DNA co-precipitation, DEAE-dextran-mediated transfection, polybrene-mediated transfection, electroporation, microinjection, liposome fusion, lipofection, protoplast fusion, retroviral infection, and biolistics. The same methods may be employed for introducing the nucleic acid molecule encoding the recombinase into the cell. Preferably, said nucleic acid molecules are contained in a vector expressible in the target cell.

Vector modification techniques are described for example in Sambrook and Russel "Molecular Cloning, A Laboratory Manual", Cold Spring Harbor Laboratory, N.Y. (2001) and in the respective manufacturer's manuals.

Non-limiting examples of vectors as used herein include plasmids, cosmids, virus, bacteriophages or other conventionally used vectors in genetic engineering. Non-limiting examples of commercially available vectors include prokaryotic plasmid vectors, such as the pUC-series, pBluescript (Stratagene), the pET-series of expression vectors (Novagen) or pCRTOPO (Invitrogen) and vectors compatible with an expression in mammalian cells like pREP (Invitrogen), pcDNA3 (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, plZD35, pLXIN, pSIR (Clontech), pIRES-EGFP (Clontech), pEAK-10 (Edge Biosystems) pTriEx-Hygro (Novagen), pEntry vectors (Invitrogen) and pCINeo (Promega). Examples for plasmid vectors suitable for Pichia pastoris comprise e.g. the plasmids pAO815, pPIC9K and pPIC3.5K (all Intvitrogen).

Generally, vectors can contain one or more origin of replication (ori) and inheritance systems for cloning or expression, one or more markers for selection in the host, e. g., antibiotic resistance, and one or more expression cassettes. Suitable origins of replication (ori) include, for example, the Col E1, the SV40 viral and the M 13 origins of replication.

Ligation of the coding sequences to transcriptional regulatory elements and/or to other amino acid encoding sequences can be carried out using established methods. Transcriptional regulatory elements (parts of an expression cassette) ensuring expression in prokaryotes or eukaryotic cells are well known to those skilled in the art. These elements comprise regulatory sequences ensuring the initiation of the transcription (e. g., translation initiation codon, promoters, enhancers, and/or insulators), T2A, P2A or similar sequences (Smyczak et al., 2004), internal ribosomal entry sites (IRES) (Owens, Proc. Natl. Acad. Sci. USA 98 (2001), 1471-1476) and optionally poly-A signals ensuring termination of transcription and stabilization of the transcript. Additional regulatory elements may include transcriptional as well as translational enhancers, and/or naturally-associated or heterologous promoter regions. Preferably, the nucleic acid molecules of the invention are operatively linked to such expression control sequences allowing expression in cells.

Possible examples for regulatory elements ensuring the initiation of transcription comprise the cytomegalovirus (CMV) promoter, SV40-promoter, RSV-promoter (Rous sarcome virus), the lacZ promoter, the gai10 promoter, human elongation factor 1α-promoter, CMV enhancer, CaM-kinase promoter, the Autographa californica multiple nuclear polyhedrosis virus (AcMNPV) polyhedral promoter or the SV40-enhancer. Examples for further regulatory elements in prokaryotes and eukaryotic cells comprise transcription termination signals, such as SV40-poly-A site or the tk-poly-A site or the SV40, lacZ and AcMNPV polyhedral polyadenylation signals, downstream of the polynucleotide.

An expression vector according to this invention is capable of directing the replication, and the expression, of the nucleic acid molecule and encoded enzyme. Suitable expression vectors which comprise the described regulatory elements are known in the art such as Okayama-Berg cDNA expression vector pcDV1 (Pharmacia), pRc/CMV, pcDNA1, pcDNA3 (In-Vitrogene, as used, inter alia in the appended examples), pSPORT1 (GIBCO BRL) or pGEMHE (Promega), or prokaryotic expression vectors, such as lambda gt11, pJOE, the pBBR1-MCS-series, pJB861, pBSMuL, pBC2, pUCPKS, pTACT1 or, preferably, the pET vector (Novagen).

The term "recombinase recognition site" is used according to the definitions provided in the art. Thus, it refers to a short nucleic acid site or sequence, that is recognized by a site-specific recombinase and which becomes the crossover region during a site-specific recombination event. Non-limiting examples of recombinase recognition site include lox sites, att sites and frt sites. The term "lox site" as used herein refers to a nucleotide sequence at which the product of the cre gene of bacteriophage P1, the Cre recombinase, can catalyze a site-specific recombination event. A variety of lox sites are known in the art, including the naturally occurring loxP, loxB, loxL and loxR, as well as a number of mutant, or variant, lox sites, such as loxP511, lox5171, loxP514, loxΔ86, loxΔ117, loxC2, loxP2, loxP3 and lox P23. The term "frt site" as used herein refers to a nucleotide sequence at which the product of the flp gene of the yeast 2 micron plasmid, FLP recombinase, can catalyze site-specific recombination. Frt sites include the naturally occurring "FRT" as well as the "F3" and "F5" site. The orientation of the recombinase recognition sites dictates one of three types of site specific recombination reactions: (i) excision, if two identical recombinase recognition sites are present in the same direction; (ii) inversion, if two identical recombinase recognition site are present in opposite direction and (iii) exchange, if two different (heterotypic) recombinase recognition sites are present in opposite or identical direction.

Also the term "recombinase" is used in accordance with the definitions provided in the art. Thus, it refers to a genetic recombination enzyme that mediates site-specific recombination in cells. Site specific recombinases are naturally occurring only in prokaryotes and lower eukaryotes. There are two classes of site-specific recombinases, tyrosine recombinases (integrases) and serine recombinases (invertases/resolvases). Tyrosine recombinases act via a nucleophilic attack by tyrosine hydroxyl. They form a covalent protein/DNA intermediate with the 3' end of the nicked strand and a holliday junction intermediate. Non-limiting examples of tyrosine recombinases include Cre recombinase from E.coli phage P1, FLP recombinase from yeast 2m episome, I integrase from E.coli I phage and XerC/XerD recombinase from E.coli. Serine recombinases act via nucleophilic attack by serine hydroxyl. They cut all four DNA strands simultaneously, form a covalent protein/DNA intermediate with the 5' end of the nicked strand and form concerted four-strand, double-stranded break intermediates. Non-limiting examples include Hin recombinase from Salmonella flagella antigen switch, gamma-delta resolvase from the Tn*1000* transposon and ΦC31 integrase from Streptomyces phage (large serine subclass).

The requirement that a recombinase recognition site is specifically recognised by a particular recombinase (such as for example the first, second, third and/or fourth recombinase) means that said recombinase recognition site is only recognised by said particular recombinase. For example, a loxP site is only recognised by Cre recombinase but not by FLP recombinase. Thus, a loxP recombinase recognition site is specifically recognised by Cre recombinase. While one recombinase recognition site can only be recognised by one recombinase it is nonetheless possible that one recombinase can recognise multiple recombinase recognition sites. For example, Cre recombinase not only recognises loxP, but also the above recited recombinase recognition sites loxB, loxL, loxR, loxP511, loxP514, loxΔ86, loxΔ117, loxC2, loxP2, loxP3 and lox P23. As site-specific recombination only occurs between matching recombinase recognition sites, the use of different recombinase recognition sites within the transgene cassette ensures that recombination only occurs between the genome and the cassette but not within the cassette.

It is further required that the first and the fourth recombinase are endogenously present in the cell or that they or corresponding nucleic acid molecules are introduced into the cell. Thus, upon introduction of the transgene cassette into the cell, site-specific recombination of the transgene cassette into the genome can occur due to the presence and activity of the recombinase. At the same time, it is required that the second and third recombinase are not endogenously present or are not active in the cell at the time of producing the cell comprising the conditionally active transgene, as these recombinases are specific for the recombinase recognition sites that are located 5' and 3' of the selection cassette. Introduction or activation of the second and third recombinase within the cell will therefore result in the removal of the selection cassette, thus enabling the conditional activation of the transgene. These recombinases may also be introduced into the cell via a nucleic acid molecule that is capable of expressing the recombinase in the target cell.

The selection cassette of step (iii) comprises a positively selectable marker gene. The term "positively selectable marker gene" is defined in accordance with the definitions provided in the art. In particular, this term refers to a gene that either encodes an enzymatic activity conferring the ability to grow in medium lacking what would otherwise be an essential nutrient or that confers upon the cell resistance to, for example, an antibiotic or drug. The expression of the selectable marker gene allows for the isolation of cells in media containing a selecting agent, such as for example neomycin, puromycin or hygromycin. For the method of the present invention, the selection marker gene has to enable positive selection, i.e. expression of the selection marker gene leads to the presence of proteins necessary for survival of the cell in an otherwise toxic culture medium. Preferably, the selection cassette is promoter-less. Thus, the selection marker gene is only expressed upon successful integration of the cassette at a position 3' of an endogenous promoter. Positive selection for clones possessing resistance to the selection marker is therefore dependent on the presence of said endogenous promoter and excludes clones in which the cassette has integrated randomly into the genome at site without a promoter. Preferably, the selection cassette further comprises a splice acceptor site located upstream of the selectable marker gene. The term "splice acceptor site" is defined in accordance with the definitions provided in the art. Thus, it refers to the 3' splice site of an intron which typically consists of a polypyrimidine tract, a branch site and a canonical or cryptic splice site, and is recognized by cellular riboprotein ccomplexes (snRPS), the Splicosome. Also preferred in accordance with the present invention is that the selection cassette further comprises a polyadenylation sequence. In accordance with the present invention, the term "polyadenylation sequence", also referred to herein as pA or poly(A) sequence herein, refers to a nucleic acid sequence that comprises the AAUAAA consensus sequence, which enables polyadenylation of a processed transcript. In general, the poly(A) sequence is located downstream of the selectable marker gene or the gene of interest and signals the end of the transcript to the RNA-polymerase.

It is further required that the genome of the cell comprises two recombinase recognition sites, i.e. a 5' recombinase recognition site and a 3' recombinase recognition site, which flank the region to be exchanged. In order to achieve integration of the transgenic cassette, the 5' recombinase recognition site is identical to one of the recombinase recognition sites of either (i) or (vi) and the 3' recombinase recognition site is identical to the other of the recombinase recognition sites of (i) or (vi). Preferably, the 5' recombinase recognition site is identical to the recombinase recognition sites of (i) and the 3' recombinase recognition site is identical to the recombinase recognition sites of (vi). In order to ensure that the targeting vector is integrated into the genome in the desired direction and that no additional inversion events happen after integration it is preferred that the 5' recombinase recognition site and a 3' recombinase recognition site of the genome are different from each other. The same applies to the corresponding recombinase recognition sites of the transgenic cassette. These recombinase recognition sites can thus be entirely unrelated sequences that are recognised by different recombinases or can be heterotypic recombinase recognition sites recognised by the same recombinase.

Furthermore, the recombinase recognition sites comprised in the genome of the cell have to be located 3' of an endogenous cellular promoter such that introduction of the transgenic cassette into the genome by site-specific recombination results in the promoter being operatively linked to the selectable marker gene and, after excision of the selection cassette, to the transgene. In the case where a transgenic cassette comprising a splice acceptor site is used, the transgenic cassette is preferably constructed such that the endogenous promoter is located within 500 bp, more preferably within 400 bp, such as for example within 300 bp and more preferably within 200 bp of the splice acceptor site. When a transgenic cassette not comprising a splice acceptor site is used, the distance to the endogenous promoter may be more than 500 bp.

The term "promoter" is used herein according to the definitions provided in the art. Thus, it refers to a DNA regulatory region capable of binding RNA polymerase in a cell and initiating transcription of a downstream (3' direction) coding sequence. Preferably, protein binding domains (consensus sequences) responsible for the binding of RNA polymerase as well as "TATA" boxes and "CAT" boxes are present within the promoter.

The term "operatively linked" as used herein refers to the requirement that the gene to be transcribed (e.g. in accordance with the method of the invention either the selection marker gene or the transgene) is inserted 3' (i.e. downstream) of the promoter such that it is under the control, i.e. becomes expressed upon activation, of the promoter leading to transcription of the gene and/or the synthesis of the corresponding protein. The term also refers to the linkage of amino acid sequences in a manner that the reading frame is maintained and a functional protein is produced. Means to achieve an operative link to the promoter include without being limiting (i) a splice acceptor and a Kozak translational start consensus sequence, (ii) an internal ribosomal entry site (IRES) and (iii) a splice acceptor and a 2A-like sequence derived from insect virus *Thosea asigna* (T2A, P2A etc. sequence). Furthermore, a nucleic acid sequence can also be operatively linked to the promoter by ensuring that the inserted sequence is in frame with the reading frame of the endogenous gene under control of said promoter. These methods are well known in the art and described, for example, in Nelson *et al.* 2008, Piccerna *et al.* 2005 or Scohy et al 2000.

Preferably, site specific recombination is achieved by recombinase mediated cassette exchange. The technique of recombinase mediated cassette exchange is well known in the art and allows the modification of higher eukaryotic genomes by targeted integration. Generally, this is achieved by the exchange of a preexisting "gene cassette" for an analogous cassette carrying the "gene of interest", as described for example in Cesari *et al.* 2004 and Cobellis et *al.* 2005 (loc. cit.).

In a further step of the method of the invention, it is required that the cell is cultured in the presence of a selection medium specific for the selectable marker encoded by the selectable marker gene of (iii). Due to the selection cassette being operatively linked to the endogenous promoter, the selectable marker gene is expressed in the cell thus enabling selection of clones having the successfully integrated transgene cassette.

In accordance with the present invention it was surprisingly found that recombinase-mediated cassette exchange (RMCE) with existing FlEx promoter traps allows the modification of an entire cell library, such as an ES cell library, in a highly efficient and straightforward manner. Typical exchange efficiencies ranged around 40% on average with a clear preference towards the first introns of genes, which is in agreement with the overall insertion preferences of splice-acceptor containing gene trap vectors in large-scale screens (Floss and Wurst, 2000; Hansen et al., 2003).

Thus, in accordance with the present invention, a powerful RMCE vector system was designed that is compatible with the established GGTC FlEx library (Schnütgen et al., 2005; Floss and Schnütgen, 2008). Whereas any existing exchange vectors can be used with the method of the invention, the examples also provide a procedure that comprises as a first step a single cloning step into a Gateway Entry vector (Invitrogen) followed by an overnight Gateway reaction to end up with the final RMCE vector. Even more easily, any cDNA, which is already situated between the attL 1/2 sites of a pEntry vector can be introduced directly into the exchange vector described here without further cloning steps. The circular vector is co-transfected along with FLP recombinase into FlEx clones, followed by hygromycin selection for 9 days. Since exchange efficiencies typically range around 40% on average, only few clones need to be picked and screened by PCR using generic primers in order to distinguish correctly exchanged alleles from randomly inserted cassettes.

While a number of studies demonstrated recently the advantages of RMCE systems in order to generate mice carrying different knock-in alleles or even "humanized mice", these studies were entirely based on "one-gene-at-a-time" approaches including tedious vector design and gene-targeting efforts (Cesari et al., 2004; Liu et al., 2006; Jaegle et al., 2007; Bateman and Wu, 2008; Sato et al., 2008). Using the method of the present invention instead allows for an easy and fast approach to target an entire library of more than 6965 conditional gene trap mutations in independent genes, among these 791 disease-related genes.

A seemingly similar RMCE system with comparable efficiencies of their exchange vector pEXCH1 was recently introduced by Cobellis et al. (2005). The exchange vector pEXCH2 however, which was designed to carry the transgenic cargo, uses a non-removable TK promoter-driven antibiotic resistance cassette as compared to the removable selection marker gene comprised in the targeting vector of the present invention. The exchange vector used by Cobellis et al. selected higher numbers of random insertions and therefore had exchange efficiencies of a lower rate (7%) when compared to pEx-Flp used in accordance with the present invention. In addition, a pEx-Flp vector as used in accordance with the present invention is Gateway-compatible and allows conditional gene expression *in vivo.* Such a conditional gene expression is not possible with the methods described in the above cited documents.

Thus, the method of the present invention extends the possibilities for further conditional gene expression, restricted only by the availability of FlEx gene trap clones. First, it represents an alternative to conventional transgenic technology with entire control over copy number and endogenous expression already *in vitro.* This should circumvent e.g. epigenetic inactivation as a result of high copy number followed by co-suppression of transgenes (Bingham et al., 1997). Second, the FlEx-RMCE method of the present invention facilitates genetic pathway dissection by allowing replacement of presumed upstream transcriptional regulators with the potential target genes. Third, this system provides a simple method in order to replace given mouse genes with the human counterpart on the transcriptional level. As is demonstrated here, it is especially suited for the expression of human disease-causing genes containing point mutations in the corresponding cells and tissues of the mouse. The GGTC FlEx library currently contains more than 791 independent confirmed or candidate genes for human genetic diseases. Fourth, this system will be particularly of interest to further extend the catalogue of Cre-expressing mouse lines.

In a preferred embodiment of the method of the invention, the first, second, third and fourth recombinase is selected from the group consisting of Cre recombinase, Flp recombinase, ΦC31 integrase, Flpe recombinase and Dre recombinase.

In accordance with the method of the present invention, each of the first, second, third and fourth recombinase can be selected from the above recited group of recombinases, such that four different recombinases are used. Alternatively, the first and fourth recombinase may be identical to each other but have to be different from the second and third recombinase. Furthermore, the second and third recombinase may be identical to each other but have to be different from the first and fourth recombinase.

The terms "Cre recombinase", "Flp recombinase", "ΦC31 integrase", "Flpe recombinase" and "Dre recombinase" are used herein according to the definitions provided in the art and include modified versions of the naturally occurring recombinase proteins, such as for example FLPo. When the cells obtained with the method of the present invention are (co-)transfected with the recombinase FLPo for expression under the control of a PGK promoter, it is preferred that at least 50 µg of the FLPo/PGK expression plasmid per 1×10⁶ cells is (co-)transfected. More preferably, at least 70 µg per 1×10⁶ cells of the FLPo/PGK expression plasmid are (co-)transfected.

In a further preferred embodiment of the method of the invention, the fourth recombinase is identical with the first recombinase.

According to this embodiment, the same recombinase may mediate recombination at the recombinase recognition sites (i) and (vi). In this case, it is preferred that these recombinase recognition sites are heterotypic recombinase recognition sites, i.e. they differ in their nucleic acid sequence but are recognised by the same recombinase. By using heterotypic recombinase recognition sites it can be ensured (i) that the targeting vector is integrated into the genome in the desired direction and (ii) that no additional inversion events happen after integration.

In a further preferred embodiment of the method of the invention, the third recombinase is identical with the second recombinase.

According to this embodiment, the same recombinase may mediate recombination at the recombinase recognition sites (ii) and (iv). In this case, the recombinase recognition sites are preferably homotypic recombinase recognition sites oriented in the same direction.

In a further preferred embodiment, the positively selectable marker is selected from the group consisting of β-lactates, glykopeptides, polyketides, aminoglykosides, polypeptide antibiotics, chinolones and sulfonamides.

All of the markers described herein are well known to the skilled person and are defined in accordance with the prior art and the common general knowledge of the skilled person. A summary is provided in von Nussbaum, F. *et al*. 2006.

β-lactames are a class of antibiotics that include penicillin derivatives (penams), cephalosporins (cephems), monobactams, and carbapenems. β-lactames share as their common structural element the four-membered azetidinone or β-lactam ring, which is their pivotal reference mark and center of action. In most β-lactame antibiotics, this central β-lactam ring is fused to a second five- or six-membered ring system. The β-lactame group of antibiotics acts by inhibiting the synthesis of the peptidoglycan layer of bacterial cell walls.

Glycopeptide antibiotics inhibit bacterial cell-wall biosynthesis by recognizing and strongly binding to the L-Lys-D-Ala-D-Ala termini of peptidoglycan precursor strands at the external side of the membrane. In this way, transpeptidases are prevented from executing their cross-linking activity.

Polyketide antibiotics, such as for example the subgroup of macrolide antibiotics, are an important class of therapeutic agents that target protein biosynthesis and act against community-acquired respiratory infections such as community-acquired pneumonia (CAP), acute bacterial exacerbations of chronic bronchitis, acute sinusitis, otitis media, and tonsillitis/pharyngitis. Non-limiting examples of polyketide antibiotics include tetracycline and erythromycin.

Aminoglykoside antibiotics cause misreading of the mRNA code and incorporation of incorrect amino acids into the peptide, thus interfering with bacterial protein biosynthesis.

Polypeptide antibiotics act on the bacterial cell membrane, where they interfere with transport mechanisms thus resulting in the bacteria being unable to excrete substances toxic to cell functions. Non-limiting examples of polypeptide antibiotica are polymyxines, bacitracin and tyrothricin.

Quinolones, also referred to as fluoroquinolones, are a family of synthetic broad-spectrum antibiotics and also includes synthetic chemotherapeutic antibacterials. They prevent bacterial DNA from unwinding and duplicating. Recent evidence has shown that topoisomerase II is also a target for a variety of quinolone-based drugs

Sulfonamides act as competitive inhibitors of the enzyme dihydropteroate synthetase, DHPS. DHPS catalyses the conversion of PABA (para-aminobenzoate) to dihydropteroate, a key step in folate synthesis. Folate is necessary for the cell to synthesize nucleic acids and in its absence cells will be unable to divide. Hence the sulfonamide antibacterials exhibit a bacteriostatic rather than bactericidal effect.

In an even more preferred embodiment, the polypeptide antibiotics marker is selected from the group consisting of chloramphenicol, tetracyclin, neomycin, hygromycin or puromycin.

Chloramphenicol is a bacteriostatic antimicrobial originally derived from the bacterium *Streptomyces venezuelae* that functions by inhibiting bacterial protein synthesis. Chloramphenicol is effective against a wide variety of Gram-positive and Gram-negative bacteria, including most anaerobic organisms.

Tetracyclines are broad-spectrum polyketide antibiotics produced by the *Streptomyces* genus of Actinobacteria and are indicated for use against many bacterial infections. Tetracyclines work by binding the 30S ribosomal subunit and through an interaction with 16S rRNA, they prevent the docking of amino-acylated tRNA. Thus, they are protein synthesis inhibitors.

Neomycin is an aminoglycoside antibiotic that blocks protein biosynthesis by binding to the the 30S ribosomal subunit.

The term "hygromycin" as used herein preferably refers to hygromycin B, which is an antibiotic produced by the bacterium *Streptomyces hygroscopicus.* It is an aminoglycoside that kills bacteria, fungi and higher eukaryotic cells by inhibiting protein synthesis.

Puromycin is an aminonucleoside antibiotic, derived from the *Streptomyces alboniger* bacterium, that causes premature chain termination during translation taking place in the ribosome. Part of the molecule resembles the 3' end of the aminoacylated tRNA. It enters the A site and transfers to the growing chain, causing premature chain release. The exact mechanism of action is unknown, but the 3' position contains an amide linkage instead of the normal ester linkage of tRNA, the amide bond makes the molecule much more resistant to hydrolysis and thus causes the ribosome to become stopped.

In another preferred embodiment of the method of the invention, the insertion of the conditional transgenic nucleic acid sequence into the target genome replaces an existing nucleic acid sequence within the target genome, wherein said existing nucleic acid sequence comprises a 5' and a 3' recombinase recognition site specifically recognised by the first and fourth recombinase of (i) and (iv).

The existing nucleic acid sequence comprising a 5' and a 3' recombinase recognition site specifically recognised by the first and fourth recombinase of (i) and (iv) can have been previously generated by homologous recombination or, alternatively, by random insertion, such as via gene trapping, transposon mutagenesis, retroviral insertion or adenoviral insertion.

In another preferred embodiment of the method of the invention, the transgene of step (v) of the method comprises a 5' splice acceptor site and a 3' poly-adenylation sequence. The presence of the splice acceptor site upstream (i.e. 5') of the transgene ensures that the transgene becomes operatively linked to the endogenous promoter in those cases where the transgene is not in frame with an exon of the endogenous gene. The presence of the 3' poly-adenylation sequence ensures termination of transcription of the transgene in those cases where the endogenous gene is inactivated by the method of the invention.

In another preferred embodiment of the method of the invention, the transgene of step (v) of the method comprises at its 5' and 3' end transposase recognition sites.

The presence of transposase recognition sites enables the "local hopping" phenomenon of Sleeping Beauty (SB) transposase as shown in the examples. Since almost half of the SB-mediated excision/reintegration events usually occur on the same chromosome, this system allows for the rapid generations of allelic series of mutations *in vitro* and *in vivo* which can be subjected to phenotypic screening.

The invention further relates to a method of producing a conditional transgenic non-human mammalian animal, the method comprising transferring a cell produced by the method of the invention into a pseudo pregnant female host.

The term "a conditional transgenic non-human mammalian animal", in accordance with the present invention, refers to a non-human mammalian animal carrying a transgene in its genome, wherein the expression of the transgene can be activated in a tissue- and time-dependent manner.

In accordance with the present invention, the term "transferring a cell produced by the method of the invention into a pseudo pregnant female host" includes the transfer of a fertilised oocyte but also the transfer of pre-implantation embryos of for example the 2-cell, 4-cell, 8-cell, 16-cell and blastocyst (70-to 100-cell) stage. Said pre-implantation embryos can be obtained by culturing the cell under appropriate conditions for it to develop into a pre-implantation embryo. Furthermore, injection or fusion of the cell with a blastocyst are appropriate methods of obtaining a pre-implantation embryo. Where the cell produced by the method of the invention is a somatic cell, derivation of induced pluripotent stem cells is required prior to transferring the cell into a female host such as for example prior to culturing the cell or injection or fusion of the cell with a pre-implantation embryo. Methods for transferring an oocyte or pre-implantation embryo to a pseudo pregnant female host are well known in the art and are, for example, described in Nagy et al., (Nagy A, Gertsenstein M, Vintersten K, Behringer R., 2003. Manipulating the Mouse Embryo. Cold Spring Harbour, New York: Cold Spring Harbour Laboratory Press).

In a more preferred embodiment, the method of producing a conditional transgenic non-human mammalian animal further comprises culturing the cell to form a pre-implantation embryo or introducing the cell into a blastocyst prior to transferring it into the pseudo pregnant female host. Methods for turing the cell to form a pre-implantation embryo or introducing the cell into a blastocyst are well known in the art and are, for example, described in Nagy et al., (Nagy A, Gertsenstein M, Vintersten K, Behringer R., 2003. Manipulating the Mouse Embryo. Cold Spring Harbour, New York: Cold Spring Harbour Laboratory Press).

The term "introducing the cell into a blastocyst" as used herein encompasses injection of the cell into a blastocyst as well as fusion of a cell with a blastocyst. Methods of introducing a cell into a blastocyst are described in the art, for example in Nagy et al., (Nagy A, Gertsenstein M, Vintersten K, Behringer R., 2003. Manipulating the Mouse Embryo. Cold Spring Harbour, New York: Cold Spring Harbour Laboratory Press).

The present invention further relates to a conditional transgenic non-human mammalian animal obtainable by the above described method of the invention.

The generation of transgenic non-human mammalian animals in accordance with the present invention has numerous applications in research in order to understand the mechanism underlying disease development and progression as well as to test potential therapeutic approaches. A few examples of possible applications are given below on the example of transgenic mice that were generated in accordance with the present invention (see examples). In these mice, a human mutant form of the TDP-43 gene carrying an A315T mutation (referred to as TDP-43(A315T) herein after) was conditionally expressed.

In humans, the presence of the mutant form of TDP-43 results in behavioural changes and personality changes, such as depression, aggression, indifferentism and disinterest, attention deficit, crossing of social boundaries, insomnia or sudden cravings for sweets. Many of these criteria could be analysed in the mouse model using specific behavioural tests such as for example the modified whole board, forced swim tests or the Morris water maze. Furthermore, disease progression in humans towards amyotrophic lateral sclerosis (ALS) and frontotemporal lobar degeneration (FTLD) is accompanied by the loss of motor neurons and, subsequently, progressive degeneration of skeletal muscle. This development could be shown in the mouse model using motoric tests such as the grip strength test.

Such animal models, or cells obtained from mutant animals, can then be used in the screening of potential pharmaceutical compositions. For example, neurons obtained from the TDP-43(A315T) mouse model can be employed to study effects of potentially therapeutically effective compounds on the subcellular distribution of TDP-43 and the potential of TDP-43 to for insoluble aggregates. Potential target drugs could target the nuclear transport mechanism of the cell or the proteasome pathway. Furthermore, such cells can be used in microarray studies in order to identify target genes of TDP-43.

So far, it is not known whether the age of patients or environmental factors such as diet, activity or the use of medical or recreational drugs have an impact on disease progression. Only smoking has been shown so far to be a risk factor in these patients. It is currently suggested that electrical injury, toxin exposure or other traumata may be further risk factors. Another theory is that a secondary mutation in a further gene is required for disease manifestation. All these theories can be simulated in animal models or, alternatively, in relevant cell cultures derived from these models.

Presently, the only drug having a life-extending effect for two to three months in human patients suffering from amyotrophic lateral sclerosis (ALS) and frontotemporal lobar degeneration (FTLD) is Riluzol. However, the underlying mechanism of Riluzol action is not known yet but it has been shown to involve an interaction with the N-methyl-D-aspartase (NMDA) receptor and the inhibition of glutamate release from presynaptic neurons. Using an animal model, the mechanism of Riluzol action can be elucidated and potentially optimized. Furthermore, additional neuroprotective substances such as for example IGF-1, benzodiazepine, tamoxifen, vitamin E etc. can be tested for a potential effect in disease development and progression.

Finally, the animal model may serve to test the potential of stem cell therapy by transplantation of neuronal stem cells and analysis of their effect on disease progression.

As will be appreciated by the skilled person, the above described approaches can be employed using any of the animal models currently established for research purposes.

In a preferred embodiment, the transgenic non-human mammalian animal of the invention is selected from the group consisting of transgenic rodents, dogs, pigs, cows or monkeys. Preferably, the transgenic non-human mammalian animals are animals routinely used in laboratory research.

Non-limiting examples of "rodents" are mice, rats, squirrels, chipmunks, gophers, porcupines, beavers, hamsters, gerbils, guinea pigs, degus, chinchillas, prairie dogs, and groundhogs. Preferably, the rodents are selected from the group consisting of mice and rats.

Non-limiting examples of "dogs" include members of the subspecies *canis lupus familiaris* as well as wolves, foxes, jackals, and coyotes. Preferably, the dogs are from the subspecies *canis lupus familiaris* and in particular are selected from beagles or Dobermans.

The term "primates", as used herein, refers to all monkey including for example cercopithecoid (old world monkey) or platyrrhine (new world monkey) as well as lemurs, tarsiers, apes and marmosets (Callithrix jacchus). Preferably, the primates are selected from the group consisting of marmosets as well as guenons, macaques, capuchins and squirrel monkeys.

All of the non-human mammalian animals described herein are well known to the skilled person and are taxonomically defined in accordance with the prior art and the common general knowledge of the skilled person.

The present invention further relates to a transgenic TDP-43 mouse, comprising a transgenic cassette in intron 1 of the mouse Tardbp gene, wherein the transgenic cassette comprises (i) a 5' recombinase recognition site specifically recognised by a first recombinase; followed by (iii) a selection cassette comprising a hygromycin selectable marker gene; followed by (iv) a 3' recombinase recognition site specifically recognised by a second recombinase; followed by (v) a hTDP-43 transgene; wherein the first and second recombinase is not endogenously present or is not active in the cell.

The term "a hTDP-43 transgene" as used herein refers to a nucleic acid sequence encoding a human TDP-43 gene. Preferably, the nucleic acid sequence encodes a mutant human TDP-43 gene, wherein the mutation is associated with a neurological disease such as for example frontotemporal lobar degeneration (FTLD) and familial amytrophic lateral sclerosis (ALS). As described above, transgenic mice were generated in accordance with the present invention (see examples) in which a human mutant form of the Tardbp gene carrying an A315T mutation (referred to as Tardbp ^{Ki-hTDP-43(A315T)}) is conditionally expressed upon recombinase mediated excision of the selection cassette. Such transgenic mice have numerous applications in research, as outlined above.

In a preferred embodiment, the hTDP-43 transgene is the Tardbp ^{Ki-hTDP-43(A315T)} transgene.

The figures show:

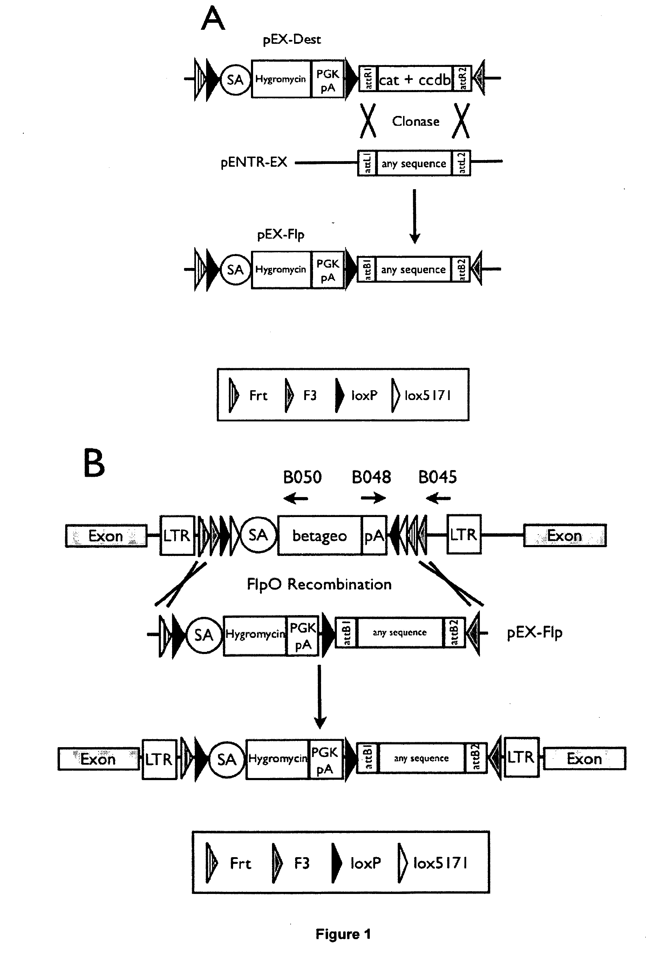

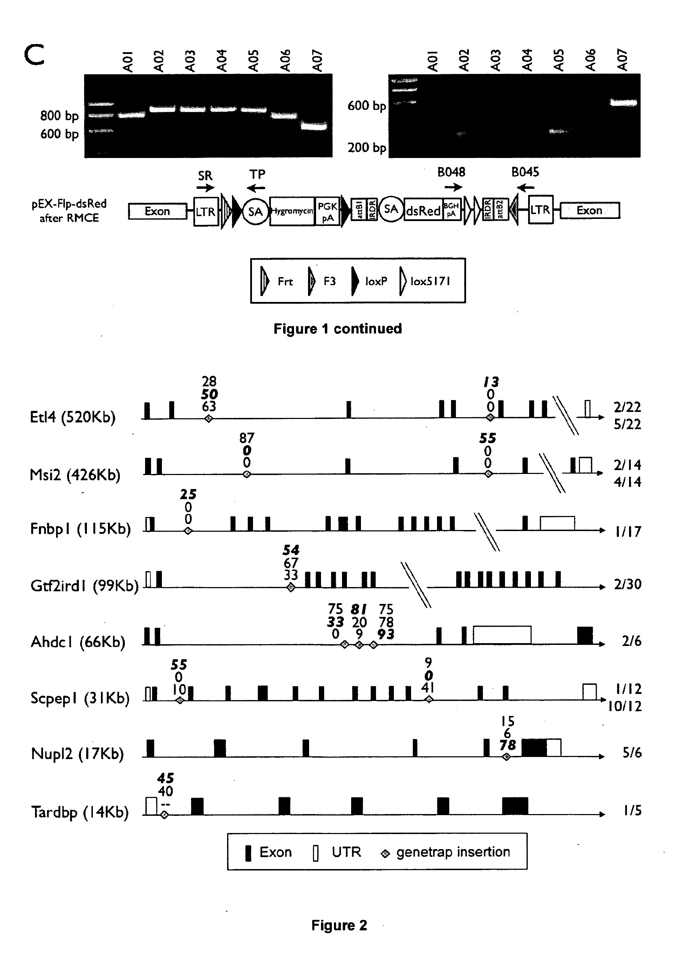

**Figure 1:** (A) Schematic outline of the procedure for individual modification and use of pEX-Flp. Any DNA sequence, which is cloned between the attL1/2 recombination sites in the pENTR-EX, can be exchanged with an attR1/2 flanked cat ccdb cassette of pEX-Dest *in vitro.* (B) Schematic illustration of the FlpO RMCE in ES cells. pEX-Flp harbors two heterospecific flippase recombination sites, therefore co-transfection of FlpO and pEX-Flp to any FlEx gene trap ES cell clone allows replacement of β-geo cassette. (C) Analysis of successfully exchanged locus with pEX-Flp containing a adSA-dsRed-BGHpA sequence flanked by IR/DR of Sleeping Beauty transposase. Example PCR screen of 8 hygromycin resistant clones (E307D01 derivates, A01-A07) concerning orientation of the genetrap vector and successful exchange (left: primers B045, B048, B050; right: primers SR, TP). A01 and A06 are inverted β-geo insertions, they show an 800 bp band on the left and no band on the right. A02-A05 are successfully exchanged clones, they show an 839 bp band on the left and the small 239 bp band on the right. Clone A07 carries the original beta-geo insertion and gives rise to the 631 bp band on the left and the 516 bp band on the right.
**Figure 2****:** Schematic overview of the efficiency of FlpO-mediated RMCE in FlEx gene trap ES cell clones. Shown are all transfected gene trap clone insertions and the corresponding rounded efficiency of exchange reaction of pEX-Flp in three different reading frames (0 upper, +1 middle, +2 lowest value in percent, red values indicating the frame matching the gene trap insertion). All gene trap clones used had insertions in an intron of the respective gene, and the ratio on the right indicates in which intron out of how many total introns the insertion is located. On the left, the size of the genes is indicated in kilobases, and the total size of the genes is not drawn in scale, with omitted DNA indicated. Untranslated and translated exons according to Ensembl. MGI Gene Identifiers and absolute expression levels are listed in Table 1.

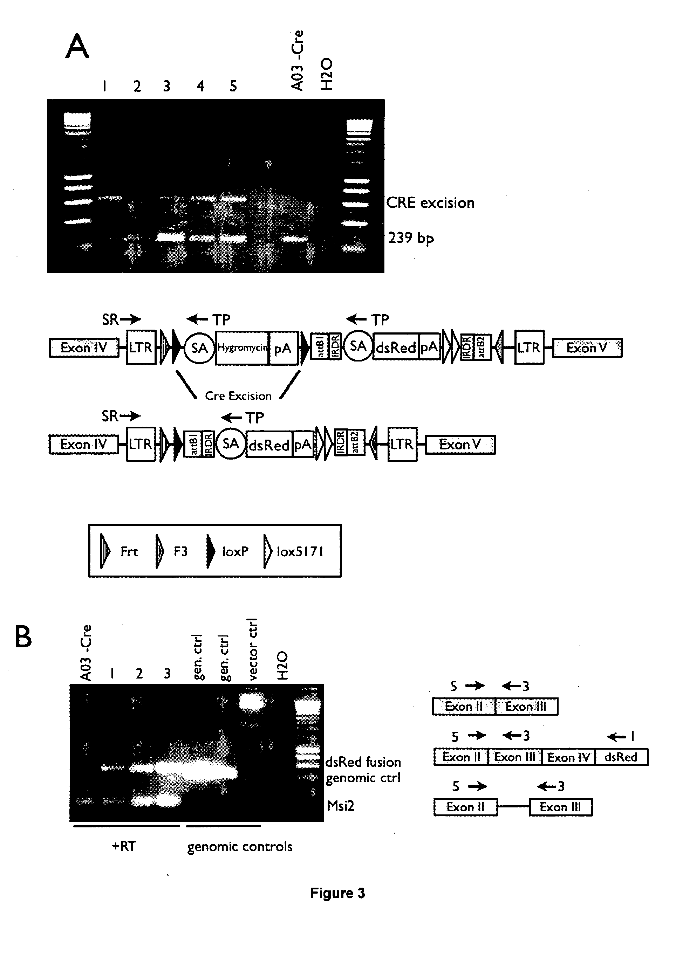

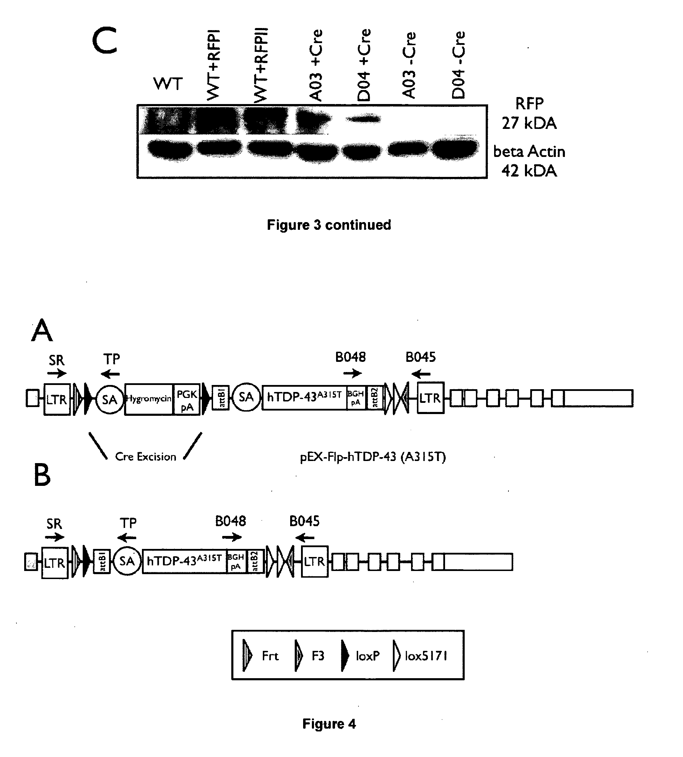

**Figure 3:** (A) Removal of hygromycin resistance upon Cre activity. Cre transfected exchange clone A03 (E307D01 derivative) was analysed using primers located within both the hygromycin/dsRed cassettes (TP) and the LTR of the original gene trap vector (SR). PCR on total genomic DNA of five pooled transfected dishes (lanes 1-5) compared to genomic DNA of clone A03 prior to Cre transfection (A03 - Cre). The internal primer (TP) gives rise to the larger product (619 bp) only after successful excision of the hygromycin cassette. Prior to hygromycin excision, primer TP amplifies a smaller product of 239 bp. (B) RT-PCR and triple PCR analysis to determine splicing to the dsRed after hygromycin excision by using external primers in exon II (5) of the insertion locus (Msi2) and an internal dsRed primer (I) and as internal wildtype control a primer located in exon III (3) of the Msi2 gene. cDNA of individual clones derived from Cre transfected clone A03 after puromycin selection (lanes labelled 1-3) compared to cDNA of clone A03 prior to Cre transfection (lane A03 -Cre). As control genomic DNA of two gene trap clones (E307D01, E326E12) and pEX-Flp plasmid were used. (C) Western blot analysis of protein extracted from ES cells probed with a polyclonal RFP antibody. As controls wild type ES cells either untransfected (WT) or transiently transfected with two different dsRed expression plasmids (WT+RFPI+II) were used. Exchange clones A03 and D04 were analysed prior (-CRE) and after (+CRE) transient CRE transfection. DsRed protein positive samples show a 27 kDa band. Lower blot shows loading control with beta actin monoclonal antibody (42 kDa).

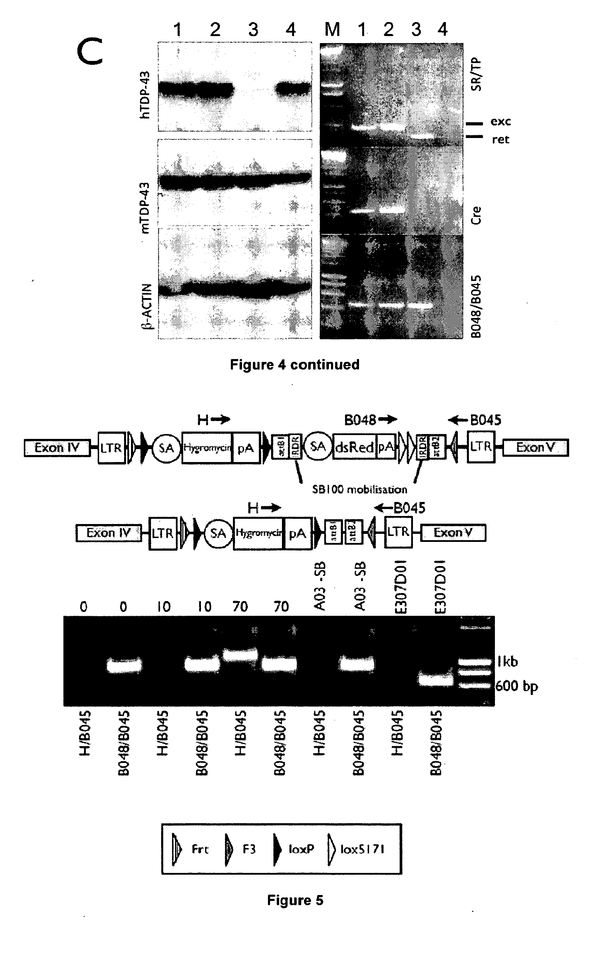

**Figure 4****:** *In vivo* excision of the hygromycin-resistance cassette (A) The gene trap vector rFLPRosabetageo, which was inserted in intron 1 of the mouse Tardbp gene (clone D045A10 in Fig. 2) was replaced by pEx-FLP-hTDP-43(A315T). A mutant mouse line with this genomic modification was established and crossed with Rosa26-Cre deleter mice. (B) The RMCE allele in the mouse Tardbp intron 1 after Cre-mediated excision of the hygromycin-resistance cassette. (C) Left: Total protein was extracted from embryonic heads, which were obtained from mating to Cre-deleter mice at E16.5. Western blots were performed using antibodies against human Tdp-43 (hTDP-43), mouse TDP-43 (mTDP-43) and b-actin. Right: DNA for genotyping was obtained from tails. PCR was performed using the primers SR/TP or B048/B045 as depicted in A. The presence of Cre was detected using primers specific for Cre-recombinase. Exc: excision (of the hygromycin-resistance cassette); Ret: retention (of the hygromycin-resistance cassette). LTR: long terminal repeat, SA: splicing acceptor, PGK-pA: phosphoglycerate kinase poly A signal; BGHpA: bovine growth hormone polyA signal; attB1/2: gateway clonase recognition sites.
**Figure 5****:** PCR analysis of SB100 transfected exchange clone to determine mobilization of IR/DR flanked cassette. Genomic DNA of pooled SB100 transfected dishes (0, 10 and 70 µg SB100 plasmid) of exchange clone A03 (E307D01/pExFLP-dsRed derivative) was screened with primers located 5' of the F3 site (B045), in the pA of dsRed (B048) and in the hygromycin-resistance cassette (H). DNA was screened with either primer combination H/B045 or B048/B045, as controls genomic DNA of clone A03 (A03 -SB) and E307D01 was used. Primers H/B045 yielded a 1 kb product only after successful excision of the IR/DR flanked cassette. Without mobilization, a 839 bp band was amplified by primers B048/B045, whereas the original gene trap insertion led to a 631 bp band.

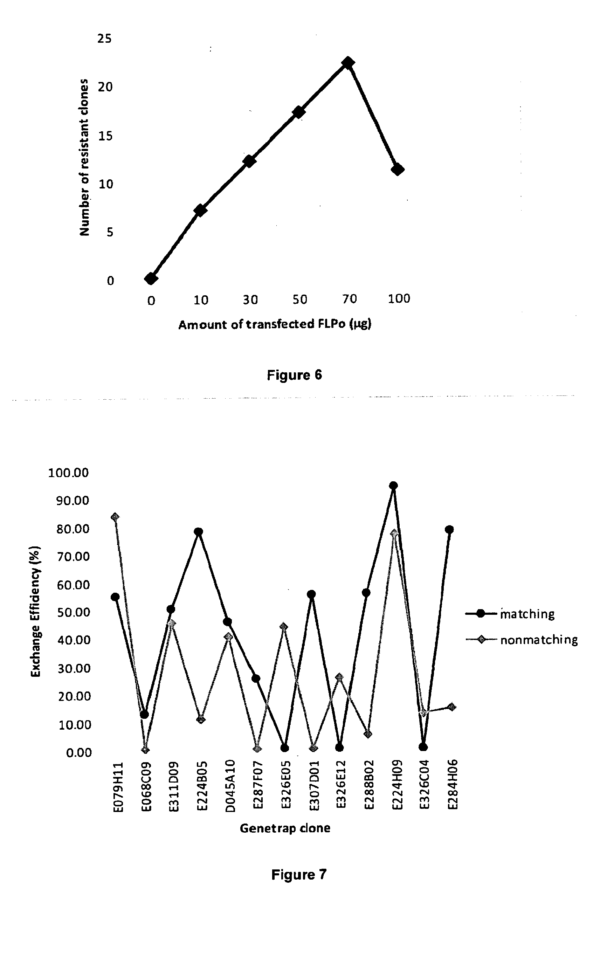

**Figure 6****:** Exchange efficiency depends on the amount of FLPo plasmid. Shown is the percentage of hygromycin resistant E307D01-derived clones after transfection of 30 µg exchange vector (pEX-Flp-dsRed) and variable amounts of FLPo expression plasmid (0, 10, 30, 50, 70 and 100 µg). The optimal amount of supercoiled FLPo plasmid to obtain the highest number of resistant clones was 70 µg.
**Figure 7****:** Differences in exchange efficiency according to matching versus nonmatching frames of the exchange vector pEX-Flp-dsRed and the gene trap vector insertion. Shown ist the exchange efficiency of each transfected gene trap clone either in the matching or nonmatching frame (average value of both nonmatching vectors). The values for clones electroporated twice were averaged likewise.

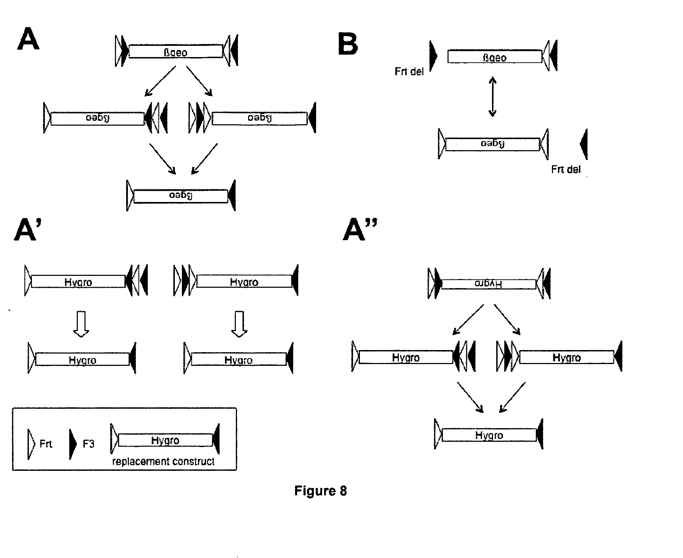

**Figure 8:** (A) Theoretical inversions of the betageo insert prior to the exchange reaction. Recombination between either Frt or F3 sites leads to transient and, after FlEx-excision, stable inversion of betageo. RMCE using the "outer" Frt/F3 sites at either stage will result in correct 5'-3' insertions of the hygromycin-resistance cassette and hence, hygromycin resistance. (B) In the event of a deleted 5' Frt site, betageo will continue to rotate in the presence of FLP recombinase. If RMCE occurs in the upper case, the hygromycin-resistance cassette will insert in inverted orientation but is able to reinvert in the presence of FLP recombinase. If the RMCE occurs in the lower case, the hygromycin insertion is in correct orientation and stable, since the 3' Frt site is lost during the recombination process. (A') The possibilities after RMCE of the replacement construct and transient states using the "inner" F3 or Frt sites. Each of the possibilities will result in hygromycin resistance before or after further FlEx excision, depending on the availability of FLP recombinase only. (A") Recombination between the "Inner" Frt/F3 sites of FlEx gene traps. In this case, hygromycin will insert in inverse orientation initially and result in hygromycin sensitivity. Depending on the availability of FLP, further inversion and excision will lead to hygromycin resistance. Note: "Hygromycin" here represents an exchange vector and "b-geo" here represents a FlEx gene trap cassette.

The examples illustrate the invention.

### Example 1: Materials and Methods

### a) Vectors

### Exchange vectors pEX-Dest and pEx-Flp

A pre-gateway pEX-Flp backbone, designated as pEx-Dest, consisting of an adenoviral splice acceptor sequence (in the following referred to as SA), followed by unique *Xhol, Sa*/*l* and *Not*l restriction sites, flanked by two identically oriented loxP sites and finally by inversely oriented 5'-FRT and 3'-F3 sites was synthesized in accordance with manufacturer's instructions (www.geneart.com) and served as a backbone for further cloning.

The hygromycin coding sequence followed by a Phosphoglycerate kinase (PGK)-polyA signal was PCR-amplified using a set of primers with a 5'-overhang containing the *Not*l restriction sites and AT-spacer nucleotides:
5'-ATGCGGCCGCGCCACCATGAAAAAGCCTGA-3',
5'-ATGCGGCCGCAAGCTTCTGATGGAATTAGA-3',
   using pPGK-Hygromycin-pA as a template. After T/A-cloning in the pCR II-Topo vector, the fragment was cloned into the *Not*l site of pEX-Dest.

Between the 3' loxP and the F3 site, a unique Pmel restriction site was utilized to insert a blunt-end Gateway cassette A (Invitrogen) containing an attR 1/2 flanked ccdB cassette (Bahassi *et al.,* 1995) to finalize pEx-Dest (Fig.1).

### Entry vector pENTR-EX

The basis for pENTR-EX was a pENTR4 insert (Invitrogen) which contains attL1/2 sites flanking the ccdB cassette (see Fig. 1A). The coding sequence of the red fluorescence protein (dsRed, from *Discosoma sp.)* was PCR-amplified from DsRed2-N1 (Clontech, Yarbrough et al., 2001) using the oligonucleotides:
5'-ATAAGCTTACCATGGCCTCCTCCGAGGAC-3' (fwd),
5'-ATGAGCTCCTACAGGAACAGGTGGTGGCG-3' (rev)
and cloned into a pCRII Topo plasmid.

Next, the pCRII Topo-dsRed *Hind*III/Sacl fragment was ligated to *HindIII*/*Sa*/l and Sacl/Sa/l fragments of the β-geo gene trap vector (adenoviral splice acceptor, β-gaiactosidase/neomycin phosphotransferase fusion gene, bovine growth hormone polyadenylation sequence, a gift of Phil Soriano to T. F.) to yield an SA-dsRed-BGHpA harbouring vector.

The pENTR4 insert and a plasmid containing a *Sleeping Beauty* (SB) transposon (PGK-Neomycin-pA flanked by SB inverted and direct repeats (IR/DR), the transposase recognition sites) were cut each by EcoRl/Sa/l and ligated. Then the *Sa*/l and the *Notl* restriction sites of the ligation product were removed, by digesting with the respective enzymes, overhangs were blunted by T4 DNA polymerase and the vector was re-ligated.

The insert flanked by the IR/DR sequences was obtained after *Hind*III digest, to ligate the resulting cassette with a *Hind*III fragment of a pEx-Flp-synthetic backbone (www.geneart.com), containing a *Sa*/*l* site, followed by two unique oriented 1o×5171 sites, which flank unique *Not*l and Ascl restriction sites.

Next, an SA-dsRed-pA sequence was released by Xhol and ligated to the attL1/2 sites of the pENTR-EX vector, linearized with *Sa*/l*.* A hyperactive *Sleeping Beauty* transposase expression plasmid (Caggs-SB100X-pA); is under control of a chicken-beta actin-CMV enhancer (Caggs) promoter (Mátés et al., 2009). A codon-optimized FLP recombinase (PGK-FLPo-pA; Raymond and Soriano 2007) was a gift from Phil Soriano to F.S. Caggs-CRE-IRES-Puro plasmid was described (Schnütgen et al. 2006).

### Gateway reaction

The final exchange vector pEx-Flp was established from pENTR-Ex and pEX-Dest in a Gateway reaction using clonase and following standard protocols (Invitrogen).

### Gene trap Insertions

Besides Tardbp (Fig. 5) and Gtf2ird, gene trap clones with insertions of rFlpRosabetageo or rsFRosabetageo were chosen randomly in either 0, +1 or +2 reading frames. rsFRosabetageo consists of a splice acceptor β-geo polyA cassette, flanked on each side by FRT, F3, loxP and lox5171 or lox511, in head-to-head orientation (FlEx array, Fig. 1B). For further details see http://www.genetrap.de as well as for example Schnütgen *et al.,* 2005.

### b) ES cell culture

### Gene trap clones

Transfections were performed using the FlEx conditional GGTC gene trap clones (for further details see http://www.genetrap.de as well as Schnütgen *et al.,* 2005):
E079H11, E068C09, E311D09, E224B05, D045A10, E287F07, E326E05, E307D01, E326E12, E288B02, E224H09, E326E04 and E284H06.

Insertions were determined by splinkerette (splk)-PCR (Horn et al., 2007) and confirmed by genomic PCR using the following primers:
E311 D09 (Etl4): 5'-gccggaagagatgctgagtc-3', 5'-tacccgtgtatccaataaaccc-3'
E068C09 (Etl4): 5'-aaactggttttcattggggatca-3', 5'-tacccgtgtatccaataaaccc-3'
E326E05(Msi2): 5'-tcccccatgtttctgtaattgg-3', 5'-gccaaacctacaggtgggtcttt-3'
E307D01 (Msi2): see hygromycin excision.
E287F07 (Fnbp1): ß-geo insertion not confirmed.
E079H11 (Gtf2irdl 5'-atcgaatgtagcccaggatg-3', 5'-gccaaacctacaggtgggtcttt-3'
E326C04 (Ahdc1): 5'-catcttgaacctcaagtttgccttt-3', 5'-tacccgtgtatccaataaaccc-3'
E284H06 (Ahdc1): 5'-ctggcttcctcccacttgtgtt-3', 5'-tacccgtgtatccaataaaccc-3'
E224H09 (Ahdc1): 5'-agaggtgaccctgctggaaatg-3', 5'-tacccgtgtatccaataaaccc-3'
E288B02(Scpep1): 5'-ccaaggtgggaaagatgaggtg-3', 5'-gccaaacctacaggtgggtcttt-3'
E326E12 (Scpep1): 5'-cacatggtgaccttcagagcag-3', 5'-gccaaacctacaggtgggtcttt-3'
D045A10 (Tardbp): 5'-acaggctaccgtatttcgtaaccaa-3', 5'-gctagcttgccaaatacaggtgg-3'
All gene trap clones are deposited in the Ensembl and NCBI genomic database.

### RMCE

All clones were derived from feeder independent E14 Tg2A.4 cells (a gift from Kent Lloyd to T. F.) gene trap lines, with the exception of D045A10 which was derived from a TBV2 cell line (Wiles et al., 2000) and was cultured on mouse embryonic fibroblast (MEF) feeder layer. ES cell lines were grown under standard culture conditions (http://www.genetrap.de). Cells were co-electroporated with 30 µg supercoiled plasmid DNA of pEX-Flp and 70µg of FLPo and selected for hygromycin resistance after 48 hours (150 µg/ml, Sigma-Aldrich) for at least 9 days.
10⁷ cells were electroporated per experiment with an EPI 2500 Elektroporations-Impulsgenerator 0-2500 V (Fischer), cells were pulsed for 2 ms at 300V in 0,4cm cuvettes in 700µl phosphate-buffered saline (PBS). After electroporation, ES cells were plated on gelatine-coated culture dishes (5 x 10⁶ cells/100 mm).
Hygromycin resistant colonies were transferred to 96-well dishes and expanded to 3 replicates of 48-wells. One plate was frozen as a stock, one was used to determine β-gal activity and one to isolate genomic DNA for further analysis.

### Cre transfection

One exchange clone (E307D01 derivative) was transfected with 50 µg of supercoiled Caggs-CRE-IRES-Puro plasmid. After electroporation, 50% of electroporated cells were plated on 5x100 mm gelatine coated dishes and cultured for 48 hrs without selection. Pooled genomic DNA served as a template for PCR analysis. The remaining cells of the electroporation were plated on a 1×100 mm gelatine coated dish and puromycin-selected for 5 days (1µg/ml, Sigma). After expansion, total RNA was extracted (Trizol) and subsequent RT-PCR was performed.

### Sleeping Beauty transfection

One exchanged clone (E307D01 derivative) was transfected with variable amounts of supercoiled SB plasmid (0, 10 and 70 µg). After plating each electroporation on 1×100mm gelatine coated dishes, cells were cultured for 48 hrs and genomic DNA was extracted of each pooled dish for PCR analysis.

### Blastocyst injection

Two successfully exchanged clones (Tardbp and Gtf2ird1) were injected into C57BI/6 host blastocysts after superovulation, in order to determine germline transmission capacities. Both clones yielded high-percentage chimeras and germline transmission.

### c) Analysis of Exchange clones

### 5' FRT site PCR

Prior to cassette exchanges, the integrity of 5' FRT sites was determined by PCR using the oligonucleotides: 5'-gccaaacctacaggtggggtcttt-3' and 5'- tgtaaaacgacgggatccgcc-3' (Floss and Schnütgen, 2008). Loss of a 5' FRT site yields a 548 bp product instead of a 673 bp product.

### X-gal staining

Initially lacZ-positive gene trap clones should become lacZ-negative after successful cassette exchange or gene trap inversion, therefore by β-gal activity false positive clones can be excluded. X-Gal staining was performed as described (Uez et al. 2008).

### Southern blot analysis

To evaluate copy number and possible random insertions after RMCE, genomic DNA of exchanged clones was analysed in a Southern blot analysis using a 800 bp neomycin (Pst I fragment of pPKJ; McBurney et al., 1991) and a 727 bp hygromycin (EcoR I/Sca I fragment of pEX-Flp) probe. DNA from 151 independent clones (90×E224B05, 12×E288B02 and 10xE326E12 derivates) was digested overnight at 37°C with Hind III for both probes and run on 0.8% agarose gel in 1×TAE. Gels were blotted on a nylon membrane (Hybond N) and hybridized following standard procedures.

### FLP inversion PCR

Conditional gene trap insertions are flanked by FlEx cassettes. Therefore FLPo also inverted the β-geo gene trap vector, which resulted in false negative β-gal activity. To analyse all RMCE clones a multiplex PCR was performed, which yielded either the inversion band of about 800 bp or the original band of 630 bp using following oligonucleotides: 5'- ctccgcctcctcttcctccat-3' (B045), 5'-cctcccccgtgccttccttgac-3' (B048), 5'-tttgaggggacgacgacagtat-3' (B050). Correctly exchanged clones produce a 839 bp fragment in this assay (Fig. 1C).

### Exchange PCR

Clones were screened for successful exchange by using an internal oligonucleotide in the splice acceptor of pEX-Flp and one in the 5'-LTR: 5'-gccaaacctacaggtggggtcttt-3' (SR), 5'-atcaaggaaaccctggactactg-3' (TP). Using these primers, positive exchange resulted in a 239 bp band, no exchange yielded a 631 bp PCR product.

### d) Further Analysis

### Hygromycin excision by Cre

This experiment was performed with the exchange clone A03, a derivative from gene trap clone E307D01. To analyse Cre mediated excision, DNA of 5 pooled transfected dishes (50µg Caggs-Cre-IRES-Puro) compared to the original exchange clone was extracted and screened by PCR for hygromycin excision using following oligonucleotides: 5'-atcaaggaaaccctggactactg-3' (TP) and 5'-gccaaacctacaggtggggtcttt-3' (SR). Undeleted hygromycin cassette led to a 239 bp product, after successful deletion a 619 bp product was amplified.

To analyse the resulting dsRed expression after Cre-mediated deletion under control of the endogenous promoter, RNA of three individual clones and the original exchange clone was extracted and analysed in a RT PCR followed in a triplex PCR. Three oligonucleotides were designed, one situated in Msi2 exon II (5'-aatgtttatcggtggactgagc-3'), one in Msi2 exon III (5'-cgtttcgttgtgggatctct-3') and an internal primer in the dsRed cassette (5'-gtgcttcacgtacaccttggag-3'). This PCR yielded a 427 bp product for the wildtype genomic sequence, a 550 bp fragment after successful Cre excision and a 94 bp fragment of the wildtype transcript.

*In vivo* excision of hygromycin by Cre was done using Rosa26Cre transgenic mice (Taconic; 006467-T-F Heterozygous C57BU6NTac-^{Gt(ROSA)26Sortm16(cre)Arte)}. Transgenic Cre mice were mated to pEx-Flp-hTDP-43 mutants and embryos were sacrificed at E17.5. Genomic DNA was isolated from tails and genotyping was done using SR/TP and B048/B045 primer combinations and Cre-specific primers pCre1 5'-atgcccaagaagaagaggaaggt-3' and pCre2 5'-gaaatcagtgcgttcgaacgctaga-3'. Undeleted hygromycin produced a band of 262bp, deletion of hygromycin led to a slightly larger fragment of 321bp. The product for Cre-specific primers was 447bp. For pEx-Flp-hTDP-43, the SR/TP and B048/B045 PCR product sizes after hygromycin deletion differed in size from all other clones tested. The reason for this was, that the SB-transposase IR/DR recognition sites, were absent from the pEx-Flp-hTDP-43 vector (Fig. 4).

### Western Blot Analysis

Isolated protein of ES cells (in RIPA buffer) were run on Nu-PAGE 10% Bis-Tris gel (Invitrogen), transferred onto PVDF membrane (Pall Corporation) and probed with polyclonal rabbit anti RFP antibody (Abcam, 1:5000 dilution) or monoclonal mouse anti beta actin (Biozol, 1:5000). The secondary antibody was peroxidase-conjugated goat anti-rabbit (Jackson Immuno Research Laboratories, INC., 1:10000 dilution) or goat anti-mouse (Jackson Immuno Research Laboratories, INC., 1:10000). For signal detection, ECL Detection Reagents I+II (GE Healthcare UK Limited) was used in conjunction with Amersham Hyperfilm ECL. TARDBP polyclonal antibody: Proteintech Group, Inc.: Purified rabbit anti human TARDBP polyclonal Antibody (dilution: 1:1500). TARDBP monoclonal antibody: Anti-human TARDBP antibody (ab57105; ABCAM; 1.25 µg/ml).

### Transposase remobilisation by SB100

This experiment was performed using exchange clone A03 (E307D01 derivative) and transfection with different amounts of SB plasmid (0, 10 and 70 µg). To analyse mobilisation of the dsRed cassette by the SB100 transposase, two PCRs with one 3' external reverse oligonucleotide (B045), and two different forward primers, either in the BGHpA of the dsRed cassette (B048), or in the hygromycin coding sequence (H) were performed. Mobilisation events led to a 1009 bp product with H/B045 combination in addition to the 839 bp product (B048/B045). Oligonucleotide sequences are:
5'-caagctctgatagagttggtcaag-3' (H),
5'-cctcccccgtgccttccttgac-3' (B048), and
5'-ctccgcctcctcttcctccat-3' (B045).

### 2. Results

### The vectors

Any given DNA can be introduced into pEX-Dest via the Gateway (Invitrogen) system; therefore the RMCE system introduced here can be applied universally. For this purpose it was designed as a two-component vector system, consisting of pEX-Dest, harboring an *in vitro* selection marker and all necessary recombinase recognition sites and the shuttle vector pENTR-EX. Both components harbor the corresponding attL/R sites to insert any sequence of interest into the final pEX-Flp in a Gateway reaction. Instead of pENTR-EX, any Gateway-compatible entry vector could be used (Fig. 1A).

### pENTR-EX

The vector pENTR4 (Invitrogen) served as a source for attL1/2 recombination sites in pEX-Flp. The cat/ccdb cassette was replaced by the IR/DR flanked adSA-dsRed-BGHpA-lox5171-lox5171 fragment (Fig. 1 C). The features of the inserted cassette are (i) inverted and direct repeats (IR/DR) which are recognized by the SB transposase; (ii) an adenoviral splice acceptor sequence (SA) followed by the promoter-less coding sequence of the red fluorescence protein (dsRed); (iii) the Bovine Growth Hormone poly(A) sequence (BGHpA); (iv) two identically oriented lox5171 sites which flank unique cloning sites, for later removal of an additional cassette, if needed. Only in the case of D045A10 (insertion in Tardbp), the cat/ccdb cassette was replaced by an ade-2 SA-flanked human cDNA (a gift of Manuela Neumann) carrying an A315T mutation (Gitcho et al., 2008) followed by BGHpA (Fig. 4).

### pEX-Dest

The vector was designed for FLP-mediated RMCE with FlEx gene trap vectors (Fig. 1 A, Schnütgen et al. 2005). The features of the pEX-Dest are (i) the face-to-face oriented 5' FRT and 3' F3 recombination sites which flank all other functional parts; (ii) the adSA followed by the hygromycin resistance gene; (iii) two head-to-tail oriented loxP sites flanking the hygromycin selection cassette for Cre-mediated excision *in vitro* or *in vivo;* (iv) the PGK poly(A) signal; (v) the clonase recombination sites attR1/2 to enable the insertion of the pENTR-EX insert.

### RMCE using FlEx gene trap clones

All gene trap clones presented herein had insertions of a retroviral SA-β-geo-pA vector, flanked by recombinase target sites in FlEx configuration (Schnütgen et al., 2005, see Fig. 1B). In brief, the cassette consists of a combination of inversely oriented original and mutant target sites for FLP and Cre recombinases. This configuration allows unidirectional inversion of the β-geo cassette by the respective recombinase. Since the pEX-Flp insert is flanked with one set of heterotypic oppositely oriented target sites for FLP recombinase, transient co-transfection of pEX-Flp and FLPo into FlEx gene trap clones allows recombination between the homotypic RRS. The RMCE strategy is outlined in Figure 1B.

Different outcomes after co-transfection of pEX-Flp and FLPo and hygromycin selection are possible: (i) recombination of pEX-Flp and β-geo in identical orientation; (ii) recombination of pEX-Flp in inverse orientation, resulting in hygromycin sensitivity. Inverse orientation is the result of recombination with the "inner" FRT/F3 sites. This event leads to hygromycin sensitivity and is therefore usually not selected. It could be followed by another inversion and excision event, leading to correctly exchanged clones; (iii) inversion of the β-geo cassette combined with a random insertion of the pEX-Flp within a transcriptionally active gene, leading to false positive clones (see Fig. 8 for a comprehensive description of all possible events before and after RMCE).

Different PCR strategies were employed to identify successfully exchanged clones. Hygromycin resistant clones were first analysed for β-gal activity. β-gal-negative clones were further screened by "exchange PCR" using a 5' primer in the LTR (SR) and a nested primer in the splice acceptor (TP), which yielded either the hygromycin band of 239 bp (A02-A05), the β-geo band of 516 bp (A07) or no band after β-geo inversion (A01; Fig. 1C right). To distinguish inverted β-geo cassettes with random insertions of pEX-Flp and to identify false positive clones as described above, a triplex PCR using primers B045, B048 and B050 was performed which yielded different product sizes depending on the orientation of the gene trap vector (Schnütgen et al., 2005). DNA from RMCE clones yielded a slightly larger product (839 bp; A02-A05) as compared to the β-geo inversion (800 bp; A01, A06), whereas original β-geo insertions led to a 631 bp product (A07; Fig. 1C, left). An assortment of clones were additionally screened by Southern blot analysis with lacZ, neomycin and hygromycin probes to detect possible multiple or random insertions. More than 90% of successfully exchanged clones showed an expected a loss of lacZ and neomycin (data not shown).

A total of 13 different conditional gene trap clones with insertions in 8 independent genes were co-transfected with pEX-Flp in three reading frames (see Fig. 2). The Scpep1 clones were electroporated twice and results were averaged.

Insertions in the same gene were either chosen for different gene trap vector reading frames or different introns (Ahdc1, Msi2, Etl4, Scpep1), to determine possible differences in exchange efficiencies between 5' and 3' insertions in the same gene and between different reading frames of pEX-Flp. Cell number and DNA amount per electroporation were identical in all cases to obtain comparable results. The efficiencies of RMCE were calculated by relating the numbers of correctly exchanged clones to the total number clones isolated per electroporation. Of the gene trap insertions used for exchange reactions, seven were in frame 0, four in frame +1 and two in frame +2, ten in more 5' introns and 3 in more 3' introns of the respective gene (Fig. 2).

Total clone number of all 38 electroporations varied from less than 10 clones in 17 cases up to several hundred clones per plate, the average clone number per electroporation was 124. The exchange efficiency of the matching frame varied from 0 to 93% and was 40% on average. The efficiency of both nonmatching frames per clone varied between 0 and 100%, but the average efficiency was only 25%. In 8 out of the 13 different electroporated genetrap clones, the matching frame was most efficient, while in 5 cases a nonmatching frame led to successful exchange (for a comprehensive comparison between the efficiencies of matching vs. non-matching frames see Fig. 7).

In five cases (Etl4 3', Fnbp1, Scpep1 5' and both Msi2 insertions), only one electroporated pEX-Flp frame led to hygromycin resistant clones, and in four out of these, it represented a frame matching the β-geo insertion. The only exception was the Msi2 insertion in intron 2, where only frame 0 yielded positive clones, while the β-geo insertion was in frame +1 (Fig. 2).

### In vitro excision of selection marker

All further screens were performed using the successfully exchanged clone A03 (Fig. 1C) derived from the E307D01 (Msi2, intron 4) gene trap clone.

The selection marker of pEX-Flp vector was designed to be removable by Cre recombinase *in vitro* or *in vivo.* To demonstrate excision *in vitro,* clone A03 was transiently transfected with Caggs-Cre-IRES-Puro plasmid with and without subsequent puromycin selection. Genomic DNA of plates without selection were pooled and screened by PCR with primers located within the splice acceptor sequence (hygromycin and dsRed) and in the LTR. This PCR yielded different sized products prior to (239 bp) or after (619 bp) excision of the selection marker (Fig. 3A). Lanes marked 1-5 (pooled transfected plates) show both band sizes, whereas untransfected A03 only shows the smaller product. Without puromycin selection, Cre excision occurred only partially. To ensure the splicing to the dsRed cassette after Cre excision, the same transfection was performed followed by puromycin selection and cDNA of individual clones was screened by PCR with primers located in Msi2 exon II and an internal primer in the dsRed coding sequence (Fig. 3B). This primer set yielded a 550 bp product in three independent clones (lanes 1-3), which was sequence-verified. As an internal control, a third primer located in Msi2 exon III was added, which yielded a 94 bp product of the wildtype transcript. As further controls genomic DNA of two gene trap clones (E307D01, E326E12) and the plasmid DNA of pEX-Flp was used. Gene trap genomic DNA templates yielded the wildtype genomic product (427 bp) including the intron 3/4.

After Cre excision of the selection marker, subsequent expression of the dsRed gene was validated in a western blot analysis. Wild type ES cells, two different positive exchange clones (A03 and D04, E307D01-derived) prior to Cre transfection and two puromycin resistant clones after transient Cre transfection (A03 and D04 derived) were screened. As positive control, wild type ES cells were transiently transfected with two different dsRed expression plasmids (WC150-DsRed2N1, WC156-DsRed2N1), expanded for 3 days and total protein was extracted. No RFP was detectable prior to Cre transfection (A03 -Cre and D04 -Cre) or in wild type protein (Fig. 3C).

### Germline transmission and excision of hygromycin in vivo

In order to analyse the germline potential of exchange clones, two clones (D045A10 and E079H11) were injected into mouse blastocysts after RMCE. Both yielded high percentage chimeras and germline transmission of the mutant allele. E079H11 carries a SB transposon (Fig. 3) and is currently being bred to SB transposase expressing mice in order to take advantage of the "local hopping" phenomenon of Sleeping Beauty transposase. Since almost half of the SB-mediated excision/re-integration events occur on the same chromosome, this system allows rapid generations of allelic series of mutations in *vitro* and *in vivo* which could be subjected to phenotypic screening. As a proof-of-principle, we therefore chose to target this SB transposon to the Williams-Beuren (WBS) critical region (Gtf2ird1 in Fig. 2). With a large number of candidate genes, the aetiology of WBS is not fully understood to date (Meyer-Lindenberg et al., 2006). An allelic series of mutations within the mouse WBS locus may contribute to a better understanding of the disease.

In the case of D045A10, which carries an insertion in Tardbp, the gene encoding TDP-43 (Ou et al., 1995) the cDNA of interest was a human mutant form of TDP-43 carrying an A315T mutation, which was recently found in familial ALS patients (Gitcho et al., 2008). In order to activate the expression of the human isoform, mice were bred to Rosa26-Cre expressing animals (TaC-^{Gt(ROSA)26Sortm16(cre)Arte}; Taconic) and total protein was isolated from embryos at E17.5. As shown in Fig. 4, embryos carrying both the Cre recombinase and the exchange vector pEx-Flp ^{TDP-43_A315T} exhibit an excision of the hygromycin cassette and initiate the expression of the human mutant isoform. The human isoform was detected by monoclonal antibody (ab57105; ABCAM), which shows no cross-reaction with the mouse protein. A polyclonal antibody (Proteintech), which recognizes both the mouse and the human Tdp-43 served as a control.

### Mobilisation of dsRed by Sleeping Beauty transposase

Clone A03 (E307D01 derivate, Msi2 insertion in intron 4) was transiently transfected with different amounts (0, 10 and 70 µg) of a SB expression vector. Pooled genomic DNA of transfected plates was screened by PCR. SB100-mediated excision was analyzed by using two primer combinations. Primers were located either in the hygromycin coding sequence (H) or in the BGHpA of the dsRed (B048) cassette (which would be mobilized by SB100) and with an external primer located 5' of the F3 site (B045). Templates of plates transfected with 10 and 70 µg SB100 showed both bands, the SB-excision band with primers H/B048 of 1009 bp and the unexcised band of 839 bp with primers B048/B045, whereas the controls 0 µg and A03 without transfection (A03-SB) only yielded the 839 bp product. As further control the gene trap clone E307D01 was used as template and yielded only a 631 bp band with primers B048/B045 (Fig. 4). All PCR products were sequence-verified.

### 3. Summary

The above examples demonstrate that RMCE with existing FlEx promoter traps allows the modification of an entire cell library, such as an ES cell library, in a highly efficient and straightforward manner. Typical exchange efficiencies ranged around 40% in average with a clear preference towards the first introns of genes, which is in agreement with the overall insertion preferences of splice-acceptor containing gene trap vectors in large-scale screens (Floss and Wurst, 2000; Hansen et al., 2003).

As shown, the overall exchange efficiencies when using FLPo recombinase were directly related to the amount of FLPo recombinase employed (Fig. 6). At otherwise identical conditions, the use of FLPo amounts under 50 µg did not efficiently select correct exchange events. At FLPo plasmid amounts of greater than 50 µg, in particular of 70 µg, significant exchange efficiencies were obtained. These high amounts of FLPo may reflect the fact that a number of different events are possible when exchanging for FlEx gene traps, namely FLP-mediated inversions and excisions prior to the exchange reaction (Fig. 8). Nevertheless, the relatively high FLPo recombinase plasmid amounts applied here raised some concern about unspecific secondary integrations of pEx-Flp. In three cases (two Ahdc1 - clones; Fig. 2), 500-2080 clones were selected as hygromycin-resistant after electroporation at identical conditions, as compared to only 50-100 clones in the majority of experiments, suggesting initially the theoretical possibility of secondary pEx-Flp insertions in transcriptionally active genes. Therefore, 96 hygromycin-resistant Ahdc1 exchange clones were analysed by Southern blotting using a hygromycin probe. We found that only 2% of successfully exchanged clones carried secondary hygromycin insertions (not shown) and the overall success rate was comparable to other clones. We therefore suspected that differences in endogenous gene expression levels may account for the differing total clone numbers after RMCE. Therefore, expression levels of the trapped genes in Fig. 2 were determined according to the absolute gene expression values using a recently published Affymetrix Chip Array data set, providing quantitative information on the expression levels of 7435 ENSEMBL genes in undifferentiated E14 ESCs (Nord et al., 2007). However, no correlation with exchange efficiencies was determined (Table 1).

Correctly exchanged clones are identified in a generic PCR using primers located within hygromycin as well as in the LTR of the original gene trap vector, which is retained in the locus after exchange (Fig. 1 B). By southern blot, we found that more than 90% of successfully exchanged clones showed the expected loss of both lacZ and neomycin (data not shown).

In order to express the cDNA of interest, the loxP-flanked hygromycin resistance cassette needs to be removed. As we show for the clones E307D01 and D045A10 (Fig. 3), hygromycin was excised upon Cre activity *in vitro.* As we demonstrate for DsRed in Fig. 3C, the gene-of-interest becomes active only after Cre activity, a feature that adds conditionality to this RMCE system if tissue-specific Cre recombinases are utilized *in vivo.*

Cre removal may be performed *in vivo* in order to avoid higher passages of ES cell clones, which may result in reduced germline rates. As was demonstrate in the examples, mating of a mouse line carrying a human mutant form of Tardbp to a ubiquitously expressing Cre deleter line resulted in solid expression of the human isoform in mouse embryos after hygromycin excision (Fig. 4). These mutants represent potential mouse models for Frontotemporal Lobar Degeneration (FTLD) and familial Amytrophic Lateral Sclerosis (ALS).

In order to further analyse whether these mouse models are indeed suitable as disease models, the following non-limiting experiments can be performed:

### Analysis of the sub-cellular localisation of TDP-43(A315T) in cells obtained from mutant mice.

In humans patients, wild-type TDP-43 is normally predominantly localised in the nucleus. However, mutant TDP-43 is predominantly located in the cytoplasm and forms insoluble aggregates in the form of heterodimers with wild-type TDP-43, thus leading to a translocation of the wild-type form into the cytoplasm. This is considered one of the reasons why the mutation acts dominant in humans. As far as is known from humans patients, this occurs in neurons, including upper and lower motorneurons, as well as in the frontotemporal cortex. Analysis of the sub-cellular localisation of TDP-43(A315T) in cells can be performed, for example, by immunohistology, cell fractionating and co-immunoprecipitation.

### Analysis of protein phosphorylation.

In human patients, only the mutant form of TDP-43 is phosphorylated. Using a monoclonal antibody against this phosphorylated form of TDP-43, for example in western blotting experiments, it can be tested whether the same phosphorylation pattern occurs in mice.

### Analysis of the C-terminal fragment.

A C-terminal fragment is cleaved of the mutated form of TDP-43, but not of wild-type TDP-43. To test for a similar processing of TDP-43(A315T) in the mouse model, western blot experiments can be performed.

### Analysis of neurodegeneration.

The occurrence of neurodegeneration in the mouse model can be tested by standard methods for neuronal degeneration. In addition, an increased amount of ubiquitinylation usually precedes neurodegeneration due to activation of the proteasomal pathway. Such changes in ubiquitinylation can be detected via immunohistology as well as western blot experiments for ubiquitin and/or caspases.

Although a successful exchange reaction is expected to occur in a frame-dependent manner, it was shown herein that matching frames become less important towards the 5' end of genes (see Fig. 7 for more info). From the GGTC library, the existence of larger number of ß-geo gene traps, selected using vectors with Kozak consensus translational start sequences in different reading frames inserted in identical introns of several genes, support our view: for this analysis, only clones with a clear genomic tag were chosen. Of a total of 5220 introns trapped, 3472 were trapped in only one, 1083 in two and 665 in all three reading frames. Of the clones which were trapped in only one frame, 63% were either in the first or second intron (referred to as 5' insertion), while 84% and 87% of hits with multiple frames were in the 5' end of genes. As likely reasons for this, we suspect initiation of translation starting at the Kozak site of the hygromycin resistance cassette, especially in cases where the first exon is untranslated (e.g. Tardbp clone; Fig. 2) as well as alternative splicing. In addition, we do not rule out the presence of alternative promoters, which were recently predicted for 40-50% of all human and mouse genes (Baek et al., 2007). Therefore, fusions with endogenous peptides are possible, but cannot be predicted. In case fusions are not desired, the use of T2A cassettes will ensure the production of two independent peptides (Smyczak et al., 2004).

The first-generation of conditional gene trap vectors (Schnütgen et al., 2005) bears a 50% risk of losing a 5' Frt site, which most likely occurs during reverse transcriptase activity in packaging cells. This risk was reduced to 10% after exchange of the spacer between 5' Frt and 5' F3 site (unpublished). The loss of the 5' Frt site may lead to continuous F3/F3 recombination and therefore rotation of the original trap or - after RMCE - the replacement vector, until transient Flp recombinase expression is lost. RMCE events on inverted betageo FlEx vectors with deletions of the 5' Frt site result in non-invertable hygromycin cassttes and were therefore most likely not selected. After sequencing of 5' FlEx arrays we identified one clone (Tardbp; Fig. 2) which lacked the 5' Frt site. In this case, overall exchange efficiencies were only 40-50%.

In summary, the RMCE system presented here extends the possibilities for further conditional gene expression, restricted only by the availability of FlEx gene trap clones. First, it represents an alternative to conventional transgenic technology with entire control over copy number and endogenous expression already *in vitro.* This should circumvent e.g. epigenetic inactivation as a result of high copy number followed by cosuppression of transgenes (Bingham et al., 1997). Second, FlEx-RMCE facilitates genetic pathway dissection by allowing replacement of presumed upstream transcriptional regulators with the potential target genes. Third, this system provides a simple method in order to replace given mouse genes with the human counterpart on the transcriptional level. As was demonstrated herein, it is especially suited for the expression of human disease-causing genes containing point mutations in the corresponding cells and tissues of the mouse. The combined GGTC and EUCOMM FlEx libraries currently contain more than 791 independent confirmed or candidate genes for human genetic diseases. Fourth, this system will be particularly of interest to further extend the catalogue of Cre-expressing mouse lines.

**Table 1: Expression levels of trapped genes depicted in Fig. 2 were determined according to their absolute gene expression values using a recently published Ayffmetrix Chip Array data set, providing quantitative information on the expression levels of 7435 ENSEMBL genes in undifferentiated E14 ESCs (Nord et al., 2007). No correlation with exchange efficiencies was found.**

| **Clone name** | **Gene** | **Insertion** | **Frame** | **LacZ original** | **Expression** | **Frame pEX-Flp** | **total clones** | **picked clones** | **efficiency** |
|---|---|---|---|---|---|---|---|---|---|
| E079H11 | Gtf2ird1 | 5' (2 von 30) | 0 | blue | 62 | 0 | 27 | 11 | 54.55 |
| | | | | | | 1 | 19 | 6 | 66.67 |
| | | | | | | 2 | 7 | 3 | 100.00 |
| E068C09 | Etl4 | 3'(5 von 16) | 0 | blue | 1770 | 0 | 24 | 16 | 12.50 |
| | | | | | | 1 | 8 | 4 | 0.00 |
| | | | | | | 2 | 13 | 5 | 0.00 |
| E311D09 | Etl4 | 5'(2 von 16) | 1 | white | 1770 | 0 | 25 | 18 | 27.78 |
| | | | | | | 1 | 15 | 6 | 50.00 |
| | | | | | | 2 | 16 | 8 | 62.50 |
| E224B05 | Nupl2 | 3' (5 von 6) | 2 | white | 249 | 0 | 430 | 34 | 14.71 |
| | | | | | | 1 | 120 | 16 | 6.25 |
| | | | | | | 2 | 2080 | 40 | 77.50 |
| D045A10 | Tardbp | 5' (1 von 5) | 0 | blue | 328 | 0 | 45 | 22 | 45.45 |
| | | | | | | 1 | 22 | 10 | 40.00 |
| E287F07 | Fnbp1 | 5' (1 von 17) | 0 | blue | 165 | 0 | 40 | 4 | 25.00 |
| | | | | | | 1 | 2 | 1 | 0.00 |
| | | | | | | 2 | 4 | 2 | 0.00 |
| E326E05 | Msi2 | 5' (2 von 14) | 1 | white | 39 | 0 | 122 | 16 | 87.50 |
| | | | | | | 1 | 0 | 0 | 0.00 |
| | | | | | | 2 | 5 | 0 | 0.00 |
| E307D01 | Msi2 | 5' (4 von 14) | 0 | blue | 39 | 0 | 128 | 20 | 55.00 |
| | | | | | | 1 | 2 | 0 | 0.00 |
| | | | | | | 2 | 6 | 1 | 0.00 |
| E326E12 | Scpep1 | 3' (10 von 12) | 1 | white | 1186 | 0 | 24 | 13 | 7.69 |
| | | | | | | 1 | 5 | 1 | 0.00 |
| | | | | | | 2 | 8 | 3 | 33.33 |
| E326E12 | Scpep1 | 3' (10 von 12) | 1 | white | 1186 | 0 | 48 | 18 | 11.11 |
| | | | | | | 1 | 7 | 4 | 0.00 |
| | | | | | | 2 | 8 | 2 | 50.00 |
| E288B02 | Scpep1 | 5'(1 von 12) | 0 | white | 1186 | 0 | 95 | 32 | 68.75 |
| | | | | | | 1 | 4 | 1 | 0.00 |
| | | | | | | 2 | 5 | 2 | 0.00 |
| E288B02 | Scpep1 | 5'(1 von 12) | 0 | white | 1186 | 0 | 240 | 24 | 41.67 |
| | | | | | | 1 | 6 | 1 | 0.00 |
| | | | | | | 2 | 11 | 5 | 20.00 |
| E224H09 | Ahdc1 | 5' (2 von 6) | 2 | blue | 100 | 0 | 917 | 32 | 75.00 |
| | | | | | | 1 | 396 | 32 | 78.13 |
| | | | | | | 2 | 224 | 32 | 93.75 |
| E326C04 | Ahdc1 | 5' (2 von 6) | 1 | blue | 100 | 0 | 25 | 4 | 25.00 |
| | | | | | | 1 | 6 | 3 | 0.00 |
| | | | | | | 2 | 6 | 1 | 0.00 |
| E284H06 | Ahdc1 | 5' (2 von 6) | 0 | blue | 100 | 0 | 145 | 27 | 77.78 |
| | | | | | | 1 | 52 | 10 | 20.00 |
| | | | | | | 2 | 77 | 11 | 9.09 |

### References

Baek D, Davis C, Ewing B, Gordon D, Green P. (2007): Characterization and predictive discovery of evolutionarily conserved mammalian alternative promoters. Genome Res. 17(2):145-55.
Baer A and Bode J (2001): Coping with kinetic and thermodynamic barriers: RMCE, an efficient strategy for the targeted integration of transgenes.J.Curr Opin Biotechnol. 2001 Oct;12(5):473-80.
Bahassi EM, Salmon MA, Van Melderen L, Bernard P, Couturier M. (1995): F plasmid CcdB killer protein: ccdB gene mutants coding for non-cytotoxic proteins which retain their regulatory functions. Mol Microbiol. Mar;15(6):1031-7.
Bateman JR, Wu CT. (2008): A simple polymerase chain reaction-based method for the construction of recombinase-mediated cassette exchange donor vectors. Genetics Nov; 180(3): 1763-6.
Bingham PM. (1997): Cosuppression comes to the animals. Cell 90(3):385-7.
Branda CS, Dymecki SM. (2004): Talking about a revolution: The impact of site-specific recombinases on genetic analyses in mice.Dev Cell. Jan;6(1):7-28.
Bethke B, Sauer B. (1997): Segmental genomic replacement by Cre-mediated recombination: genotoxic stress activation of the p53 promoter in single-copy transformants.Nucleic Acids Res. Jul 15;25(14):2828-34.
Cesari F, Rennekampff V, Vintersten K, Vuong LG, Seibler J, Bode J, Wiebel FF, Nordheim A. (2004): Elk-1 knock-out mice engineered by Flp recombinase-mediated cassette exchange. Genesis Feb;38(2):87-92.
Cobellis G, Nicolaus G, lovino M, Romito A, Marra E, Barbarisi M, Sardiello M, Di Giorgio FP, lovino N, Zollo M, Ballabio A, Cortese R. (2005): Tagging genes with cassette-exchange sites. Nucleic Acids Res. Mar 1;33(4):e44.
Dymecki, S.M., 1996. Flp recombinase promotes site-specific DNA recombination in embryonic stem cells and transgenic mice. Proc. Natl. Acad. Sci. USA 93, pp. 6191-6196.
Floss T, Wurst W. (2002): Functional genomics by gene-trapping in embryonic stem cells. Methods Mol Biol. 185: 347-79.
Floss T, Schnütgen F. (2008): Conditional gene trapping using the FLEx system. Methods Mol Biol. 435:127-38.
Gitcho MA, Baloh RH, Chakraverty S, Mayo K, Norton JB, Levitch D, Hatanpaa KJ, White CL 3rd, Bigio EH, Caselli R, Baker M, Al-Lozi MT, Morris JC, Pestronk A, Rademakers R, Goate AM, Cairns NJ. (2008): TDP-43 A315T mutation in familial motor neuron disease. Ann Neurol. Apr;63(4):535-8.
Hansen J, Floss T, Van Sloun P, Fuchtbauer EM, Vauti F, Arnold HH, Schnütgen F, Wurst W, von Melchner H, Ruiz P. (2003): A large-scale, gene-driven mutagenesis approach for the functional analysis of the mouse genome. Proc Natl Acad Sci U. S.A. 100(17): 9918-22.
Hasty P, Abuin A, Bradley A. 2000. Gene targeting, principles, and practice in mammalian cells. In: Joyner AL, editor. Gene Targeting: a practical approach, 2nd ed. Oxford: Oxford University Press. p 1-35.
Horn C, Hansen J, Schnütgen F, Seisenberger C, Floss T, Irgang M, De-Zolt S, Wurst W, von Melchner H, Noppinger PR. (2007): Splinkerette PCR for more efficient characterization of gene trap events. Nat Genet. Aug;39(8):933-4. Erratum in: Nat Genet. 2007 Dec;39(12):1528.
Jägle U, Gasser JA, Müller M, Kinzel B. (2007): Conditional transgene expression mediated by the mouse beta-actin locus. Genesis. 2007 Nov;45(11):659-66.
Liu W, Xiong Y, Gossen M. (2006): Stability and homogeneity of transgene expression in isogenic cells. J Mol Med. 84(1):57-64.
Mátés, L., Chuah, M.K., Belay, E., Jerchow, B., Manoj, N., Acosta-Sanchez, A., Grzela, D.P., Schmitt, A., Becker, K., Matrai, J., Ma, L., Samara-Kuko, E., Gysemans, C., Pryputniewicz, D., Miskey, C., Fletcher, B., VandenDriessche, T., Ivics, Z. and Izsvák, Z. (2009) Molecular evolution of a novel hyperactive Sleeping Beauty transposase enables robust stable gene transfer in vertebrates. Nat Genet. (in press).
McBurney MW, L C Sutherland, C N Adra, B Leclair, M A Rudnicki, and K Jardine The mouse Pgk-1 gene promoter contains an upstream activator sequence. Nucleic Acids Res. 1991 October 25; 19(20): 5755-5761.
Meyer-Lindenberg A, Mervis CB, Berman KF. (2006): Neural mechanisms in Williams syndrome: a unique window to genetic influences on cognition and behaviour. Nat Rev Neurosci. (5):380-93.
Nagy A, Gertsenstein M, Vintersten K, Behringer R. 2003. Manipulating the Mouse Embryo, third edition ed. Cold Spring Harbour, New York: Cold Spring Harbour Laboratory Press.
Nelson LT, Rakshit S, Sun H, Wellik DM.: Generation and expression of a Hoxa11eGFP targeted allele in mice. Dev Dyn. 2008 Nov;237(11):3410-6.
Nord AS, Vranizan K, Tingley W, Zambon AC, Hanspers K, Fong LG, Hu Y, Bacchetti P, Ferrin TE, Babbitt PC, et al. Modeling insertional mutagenesis using gene length and expression in murine embryonic stem cells. PLoS ONE (2007) 2:e617.
O'Gorman, S., Fox, D.T. and Wahl, G.M., 1991. Recombinase-mediated gene activation and site-specific integration in mammalian cells. Science 251, pp. 1351-1355.
Ou SH, Wu F, Harrich D, Garcia-Martinez LF, Gaynor RB. (1995): Cloning and characterization of a novel cellular protein, TDP-43, that binds to human immunodeficiency virus type 1 TAR DNA sequence motifs.J Virol. 1995 Jun;69(6):3584-96.
Oumard A, Qiao J, Jostock T, Li J, Bode J. (2006): Recommended Method for Chromosome Exploitation: RMCE-based Cassette-exchange Systems in Animal Cell Biotechnology. Cytotechnology Mar;50(1-3):93-108.
Park TS, Galic Z, Conway AE, Lindgren A, van Handel BJ, Magnusson M, Richter L, Teitell MA, Mikkola HK, Lowry WE, Plath K, Clark AT: Derivation of primordial germ cells from human embryonic and induced pluripotent stem cells is significantly improved by coculture with human fetal gonadal cells. Stem Cells. 2009 Apr;27(4):783-95.
Piccenna L, Shen PJ, Ma S, Burazin TC, Gossen JA, Mosselman S, Bathgate RA, Gundlach AL. Localization of LGR7 gene expression in adult mouse brain using LGR7 knock-out/LacZ knock-in mice: correlation with LGR7 mRNA distribution. Ann N Y Acad Sci. 2005 May; 1041:197-204.
Raymond CS, Soriano P. (2007): High-efficiency FLP and PhiC31 site-specific recombination in mammalian cells. PLoS ONE. Jan 17;2(1):e162.
Sato T, Kawamura Y, Asai R, Amano T, Uchijima Y, Dettlaff-Swiercz DA, Offermanns S, Kurihara Y, Kurihara H. (2008): Recombinase-mediated cassette exchange reveals the selective use of Gq/G11-dependent and -independent endothelin 1/endothelin type A receptor signaling in pharyngeal arch development. Development. 2008 Feb;135(4):755-65.
Schnütgen, F., Doerflinger, N., Calleja, C., Wendling, O., Chambon, P. and Ghyselinck, N.B., 2003. A directional strategy for monitoring Cre-mediated recombination at the cellular level in the mouse. Nat. Biotechnol. 21, pp. 562-565.
Schnütgen F, De-Zolt S, Van Sloun P, Hollatz M, Floss T, Hansen J, Altschmied J, Seisenberger C, Ghyselinck NB, Ruiz P, Chambon P, Wurst W, von Melchner H. (2005): Genomewide production of multipurpose alleles for the functional analysis of the mouse genome. Proc Natl Acad Sci USA. May 17;102(20):7221-6.
Schnütgen F, Stewart AF, von Melchner H, Anastassiadis K. Engineering embryonic stem cells with recombinase systems. Methods Enzymol. 2006;420:100-36.
Schonig K, Schwenk F, Rajewsky K, and Bujard H. Stringent doxycycline dependent control of CRE recombinase in vivo. Nucleic Acids Res 30: e134, 2002.
Seibler J, Bode J. (1997): Double-reciprocal crossover mediated by FLP-recombinase: a concept and an assay. Biochemistry. Feb 18;36(7):1740-7.
Scohy S, Gabant P, Szpirer C, Szpirer J. Identification of an enhancer and an alternative promoter in the first intron of the alpha-fetoprotein gene. Nucleic Acids Res. 2000 Oct 1;28(19):3743-51.
Seibler J, Schübeler D, Fiering S, Groudine M, Bode J. (1998): DNA cassette exchange in ES cells mediated by Flp recombinase: an efficient strategy for repeated modification of tagged loci by marker-free constructs. Biochemistry May 5;37(18):6229-34.
Schlake, T. and Bode, J., 1994. Use of mutated FLP recognition target (FRT) sites for the exchange of expression cassettes at defined chromosomal loci. Biochemistry 33, pp. 12746-12751.
Szymczak AL, Workman CJ, Wang Y, Vignali KM, Dilioglou S, Vanin EF, Vignali DA. Correction of multi-gene deficiency in vivo using a single 'self-cleaving' 2A peptide-based retroviral vector. Nat Biotechnol. 2004 May;22(5):589-94.
Torres RM, Kühn R. 1997. Laboratory protocols for conditional gene targeting. Oxford: Oxford University Press. 167 p.
Truffinet V, Guglielmi L, Cogne M, and Denizot Y. The chicken beta-globin HS4 insulator is not a silver bullet to obtain copy-number dependent expression of transgenes in stable B cell transfectants. Immunol Lett 96: 303-304, 2005.
Uez N, Lickert H, Kohlhase J, de Angelis MH, Kühn R, Wurst W, Floss T. (2008): Sall4 isoforms act during proximal-distal and anterior-posterior axis formation in the mouse embryo. Genesis. Sep;46(9):463-77.
von Nussbaum, F., Brands, M., Hinzen, B., Weigand, S. and Häbich, D. (2006): Antibacterial Natural Products in Medicinal Chemistry—Exodus or Revival? Angew. Chem. Int. Ed., 45, 5072 - 5129
Wiles M.V., Vauti F., Otte J., Fuchtbauer E.M., Ruiz P., Fuchtbauer A., Arnold H.H., Lehrach H., Metz T., von Melchner H., Wurst W. (2000): Establishment of a gene-trap sequence tag library to generate mutant mice from embryonic stem cells. Nat Genet 24(1):13-4.
Yarbrough D, Wachter RM, Kallio K, Matz MV, Remington SJ. (2001): Refined crystal structure of DsRed, a red fluorescent protein from coral, at 2.0-A resolution. Proc Natl Acad Sci USA. 98(2):462-7.

## Claims

1. A method of producing a cell comprising a conditionally active transgene in its genome, the method comprising
(a) introducing into the cell a targeting vector, wherein the targeting vector comprises
(i) a 5' recombinase recognition site specifically recognised by a first recombinase, wherein the first recombinase is endogenously present in the cell or wherein the first recombinase or a nucleic acid molecule encoding said first recombinase in expressible form is introduced into the cell; followed by
(ii) a 5' recombinase recognition site specifically recognised by a second recombinase, wherein the second recombinase is not endogenously present or is not active in the cell; followed by
(iii) a selection cassette comprising a positively selectable marker gene; followed by
(iv) a 3' recombinase recognition site specifically recognised by a third recombinase, wherein the third recombinase is not endogenously present or is not active in the cell; followed by
(v) the transgene; followed by
(vi) a 3' recombinase recognition site specifically recognised by a fourth recombinase, wherein the fourth recombinase is endogenously present in the cell or wherein the fourth recombinase or a nucleic acid molecule encoding said fourth recombinase in expressible form is introduced into the cell;
wherein the genome of the cell comprises a 5' recombinase recognition site and a 3' recombinase recognition site that are identical to the recombinase recognition sites of (i) and (vi), and wherein said recombinase recognition sites comprised in the genome of the cell are located 3' of an endogenous cellular promoter such that introduction of the targeting vector into the genome by site specific recombination results in the promoter being operatively linked to the selectable marker gene; and
(b) culturing the cell in the presence of a selection medium specific for the selectable marker encoded by the selectable marker gene of (iii).

2. The method of claim 1, wherein the first, second, third and fourth recombinase is selected from the group consisting of Cre recombinase, Flp recombinase, ΦC31 integrase, Flpe recombinase and Dre recombinase.

3. The method of claim 1 or 2, wherein the fourth recombinase is identical with the first recombinase.

4. The method of any one of claims 1 to 3, wherein the third recombinase is identical with the second recombinase.

5. The method of any one of claims 1 to 4, wherein the positively selectable marker is selected from the group consisting of β-lactames, glykopeptides, polyketides, aminoglykosides, polypeptide antibiotics, quinolones and sulfonamides.

6. The method of claim 5, wherein the polypeptide antibiotics marker is selected from the group consisting of chloramphenicol, tetracyclin, neomycin, hygromycin or puromycin.

7. The method of any one of claims 1 to 6, wherein the insertion of the conditional transgenic nucleic acid sequence into the target genome replaces an existing nucleic acid sequence within the target genome, wherein said existing nucleic acid sequence comprises a 5' and a 3' recombinase recognition site specifically recognised by the first and fourth recombinase of (i) and (iv).

8. The method of any one of claims 1 to 7, wherein the transgene of (v) comprises a 5' splice acceptor site and a 3' poly-adenylation sequence.

9. The method of any one of claims 1 to 8, wherein the transgene of (v) comprises at its 5' and 3' end transposase recognition sites.

10. A method of producing a conditional transgenic non-human mammalian animal, the method comprising transferring a cell produced by the method of any one of claims 1 to 9 into a pseudo pregnant female host.

11. The method of claim 10, further comprising culturing the cell to form a pre-implantation embryo or introducing the cell into a blastocyst prior to transferring it into the pseudopregnant female host.

12. A conditional transgenic non-human mammalian animal obtainable by the method according to claim 10 or 11.

13. The method of claim 10 or 11 or the transgenic non-human mammalian animal of claim 12, wherein the transgenic non-human mammalian animal is selected from the group consisting of transgenic rodents, dogs, pigs, cows or primates.

14. A transgenic TDP-43 mouse, comprising a transgenic cassette in intron 1 of the mouse Tardbp gene, wherein the transgenic cassette comprises
(i) a 5' recombinase recognition site specifically recognised by a first recombinase; followed by
(iii) a selection cassette comprising a hygromycin selectable marker gene; followed by
(iv) a 3' recombinase recognition site specifically recognised by a second recombinase; followed by
(v) a hTDP-43 transgene;
wherein the first and second recombinase is not endogenously present or is not active in the cell.
